(19) Europäisches Patentamt
European Patent Office
Office européen des brevets

(11) EP 4 374 879 A1

(12) EUROPEAN PATENT APPLICATION
published in accordance with Art. 153(4) EPC

(43) Date of publication:
29.05.2024 Bulletin 2024/22

(21) Application number: 22845463.3

(22) Date of filing: 22.07.2022

(51) International Patent Classification (IPC):
*A61K 47/68* (2017.01) *C07K 16/28* (2006.01)
*A61K 31/357* (2006.01) *A61K 39/395* (2006.01)
*A61P 35/00* (2006.01)

(52) Cooperative Patent Classification (CPC):
**A61K 47/6803; A61K 47/68; A61K 47/6855; A61K 47/6889; C07K 16/32;** A61K 2039/505; C07K 2317/73

(86) International application number:
PCT/CN2022/107479

(87) International publication number:
WO 2023/001300 (26.01.2023 Gazette 2023/04)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 22.07.2021 CN 202110830783

(71) Applicants:
- **Shanghai Senhui Medicine Co., Ltd.**
  **Shanghai 201203 (CN)**
- **Shanghai Shengdi Pharmaceutical Co., Ltd**
  **Shanghai 201210 (CN)**
- **Shanghai Hengrui Pharmaceutical Co., Ltd.**
  **Shanghai 200245 (CN)**
- **Jiangsu Hengrui Pharmaceuticals Co., Ltd.**
  **Lianyungang, Jiangsu 222047 (CN)**

(72) Inventors:
- **SUN, Xing**
  **Shanghai 201210 (CN)**
- **YANG, Changyong**
  **Lianyungang, Jiangsu 222047 (CN)**
- **LIANG, Jindong**
  **Shanghai 200245 (CN)**
- **LIAO, Cheng**
  **Shanghai 201210 (CN)**

(74) Representative: **Regimbeau**
**20, rue de Chazelles**
**75847 Paris Cedex 17 (FR)**

Remarks:
The complete document including Reference Table(s) and the Sequence Listing(s) can be downloaded from the EPO website

# (54) DRUG CONJUGATE OF ERIBULIN DERIVATIVE

(57) A drug conjugate of an Eribulin derivative. Specifically, provided are an HER2 antibody conjugate that is formed by binding the Eribulin derivative to structural domain II of HER2, a preparation method therefor, and a pharmaceutical application thereof. The present invention further relates to a method for treating a cancer by means of administration of an antibody-drug conjugate, and a composition.

EP 4 374 879 A1

## Description

### TECHNICAL FIELD

The present disclosure relates to a drug conjugate of an eribulin derivative.

### BACKGROUND

An antibody-drug conjugate (ADC) links a monoclonal antibody or an antibody fragment to a biologically active drug via a stable chemical linker compound, fully exploiting the binding specificity of the antibody to surface antigens of normal cells and tumor cells and the high efficiency of the drug, and also avoiding the former's disadvantage of having a poor therapeutic effect, the latter's disadvantage of having serious toxic side effects, and the like. This means that the antibody-drug conjugate can bind to tumor cells precisely and has a reduced effect on normal cells compared with conventional chemotherapeutic drugs in the past.

Since the first antibody-drug conjugate, Mylotarg (gemtuzumab ozogamicin, Wyeth Pharmaceutical Co., Ltd.), was approved by the FDA in the United States for the treatment of acute myeloid leukemia in 2000, a total of 164 ADC drugs have entered the clinical phase, most (n = 100) in the clinical phase I, 46 in the clinical phase II, 7 in the clinical phase III, and 3 in the phase of BLA application. Representative third-generation antibody-drug conjugates on the market are polatuzumab vedotin (trade name Polivy, marketed in June 2019) co-developed by Genentech and Genetics, enforiumab vedotin (trade name Padcev, marketed in December 2019) co-developed by Agensys (a subsidiary corporation of Astellas) and Seattle Genetics, and fam-trastuzumab deruxtecan (trade name Enheriu) developed by Daiichi Sankyo.

Microtubules are potent filamentous cytoskeletal proteins associated with a variety of cellular functions including intracellular migration and transport, cell signaling, and maintenance of cell shape. Microtubules also play a critical role in mitotic cell division by forming the mitotic spindle required for chromosomes to divide into two daughter cells. The biological functions of microtubules in all cells are regulated in large part by their polymerization dynamics, and the polymerization of microtubules occurs by the reversible, non-covalent addition of $\alpha$ and $\beta$ tubulin dimers at both ends of microtubules. This dynamic behavior and the resulting control of microtubule length are essential for proper function of the mitotic spindle. Even minor changes in microtubule dynamics involve spindle checkpoint, inhibit cell cycle progression at mitosis, and subsequently cause cell death. Since cancer cells divide rapidly, they are generally more sensitive to compounds that bind to tubulin and disrupt their normal functions than normal cells. Therefore, tubulin inhibitors and other microtubule-targeting agents are expected to be a class of drugs for the treatment of cancer.

### SUMMARY

The present disclosure provides an antibody-drug conjugate (ADC) having a structure of formula (I) or a pharmaceutically acceptable salt or solvate thereof:

$$Ab\text{-}(L\text{-}D)_k \qquad (I)$$

wherein Ab is an HER2 antibody or an antigen-binding fragment thereof binding to a domain II of HER2,
L is a linker covalently linking Ab to D, and k is 1 to 20 (including 1, 2, 3, 4, 5, 6, 7, 8, 9, 10, 11, 12, 13, 14, 15, 16, 17, 18, 19, 20, or any value between any two values);
-D is shown as the formula below:

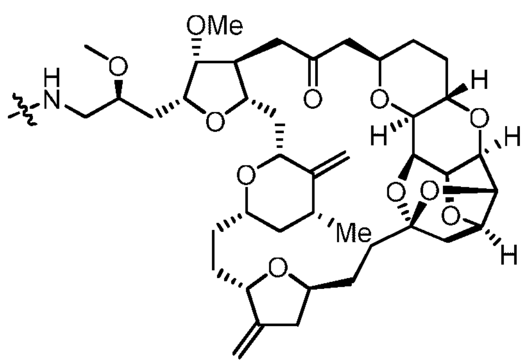

.

In some embodiments, k in the antibody-drug conjugate Ab-$(L\text{-}D)_k$ may be selected from the group consisting of 1 to 10 and can be an integer or a decimal.

In some embodiments, the linker is stable extracellularly, such that the ADC remains intact when present in an extracellular environment, but is capable of being cleaved when internalized in a cell such as a cancer cell. In some embodiments, the eribulin derivative drug moiety is cleaved from the antibody moiety when the ADC enters a cell that expresses an antigen specific to the antibody moiety of the ADC, and the cleavage releases an unmodified form of the eribulin derivative.

In some embodiments, the cleavable moiety in the linker is a cleavable peptide moiety. In some embodiments, the ADC comprising the cleavable peptide moiety shows a lower aggregation level, an improved antibody to drug ratio, increased targeted killing of cancer cells, reduced off-target killing of non-cancer cells, and/or a higher drug loading relative to the ADC comprising other cleavable moieties. In some embodiments, the addition of a cleavable moiety increases cytotoxicity and/or potency relative to a non-cleavable linker. In some embodiments, the cleavable peptide moiety is capable of being cleaved by an enzyme, and the linker is one capable of being cleaved by an enzyme. In some embodiments, the enzyme is a cathepsin, and the linker is one capable of being cleaved by a cathepsin. In certain embodiments, the enzyme-cleavable linker (e.g., the cathepsincleavable linker) exhibits one or more of the improved properties described above compared with other cleavage mechanisms.

In some embodiments, the linker comprises an amino acid unit, and the amino acid unit preferably comprises a peptide residue consisting of 2 to 7 amino acids selected from the group consisting of phenylalanine, glycine, valine, lysine, citrulline, serine, glutamic acid, and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe), and glycine-glycine-phenylalanine-glycine (Gly-Gly-Phe-Gly).

In some embodiments, the linker comprising an amino acid unit of the present disclosure is selected from the group consisting of

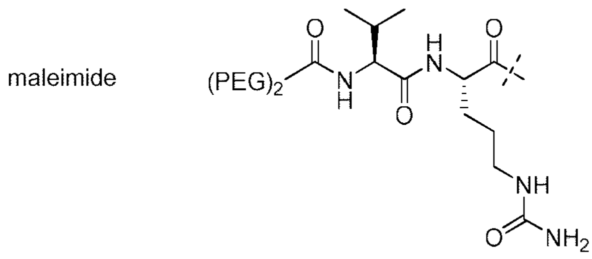

,

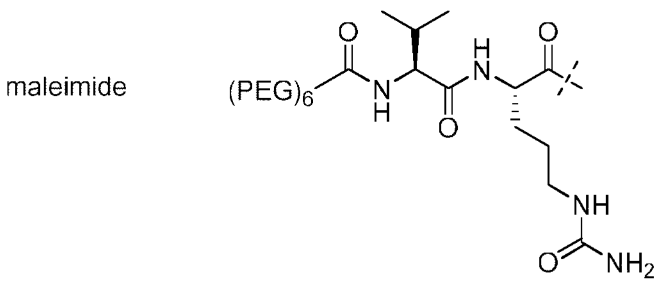

,

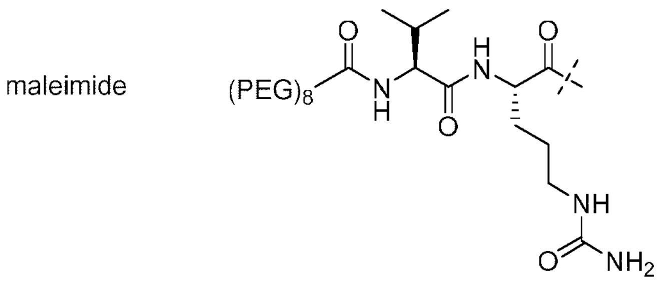

,

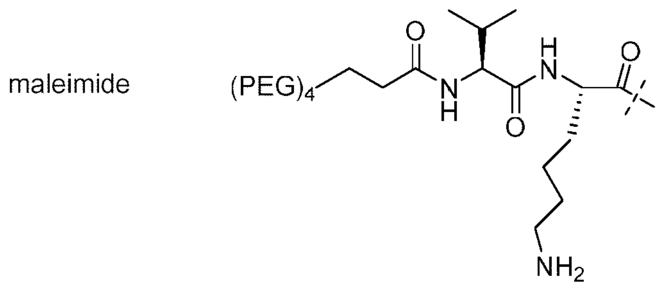

,

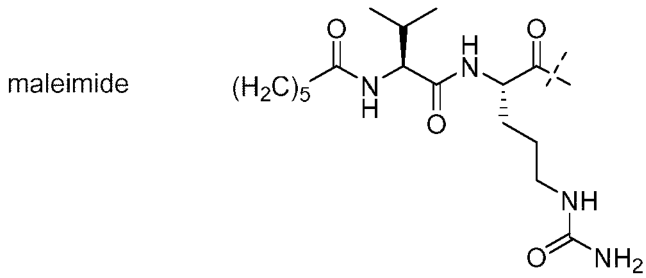

, or

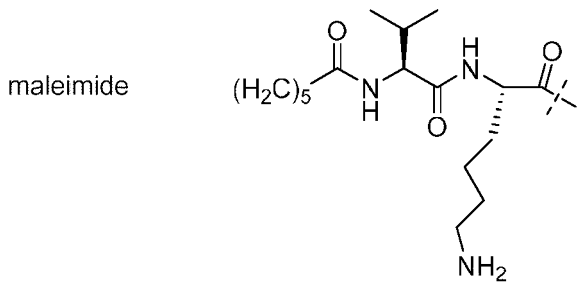

.

In some embodiments, the amino acid unit comprises valine-citrulline (Val-Cit). In some embodiments, the ADC comprising Val-Cit shows increased stability, reduced off-target cell killing, increased targeted cell killing, a lower aggregation level, and/or a higher drug loading relative to the ADC comprising other amino acid units or other cleavable moieties.

In another aspect, a linker provided by some embodiments comprises a cleavable sulfonamide moiety, and the linker is capable of being cleaved under reduced conditions. In some embodiments, the linker comprises a cleavable disulfide moiety, and the linker is capable of being cleaved under reduced conditions.

In another aspect, a linker in the antibody conjugate of the present disclosure comprises at least one spacer unit that links an eribulin derivative D to a cleavable moiety. In some embodiments, the linker comprises a spacer unit linking to D.

In some embodiments, the spacer unit comprises p-aminobenzyloxycarbonyl (pAB)

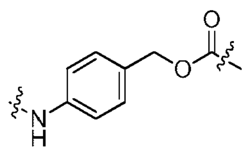

.

In some embodiments, the spacer unit comprises:

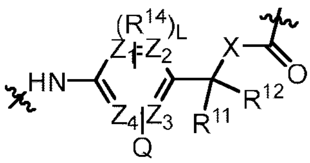

,

wherein $Z_1$-$Z_4$ are each independently selected from the group consisting of a carbon atom and a nitrogen atom; $R^4$ is selected from the group consisting of alkyl, cycloalkyl, aryl, and heteroaryl, and the alkyl, cycloalkyl, aryl, and heteroaryl are each independently optionally substituted with one or more substituents selected from the group consisting of alkyl, alkoxy, halogen, amino, cyano, nitro, hydroxy, hydroxyalkyl, cycloalkyl, heterocycloalkyl, aryl, and heteroaryl; $R^1$ and $R^2$ are each independently selected from the group consisting of hydrogen, $C_{1-6}$ alkyl, haloalkyl, and $C_{3-6}$ cycloalkyl, preferably hydrogen; or $R^1$ and $R^2$, together with the carbon atom to which they are attached, form $C_{3-6}$ cycloalkyl; X is selected from the group consisting of -O- and -NH-; L is selected from the group consisting of integers from 1 to 4;

Q is V-E, V-E provides a glycosidic bond cleavable by an intracellularly located glycosidase, and E is selected from the group consisting of -O-, -S-, and -$NR^3$-, wherein $R^3$ is selected from the group consisting of hydrogen and methyl; further, V is selected from

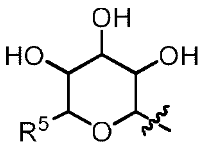

,

wherein $R^5$ is selected from the group consisting of -COOH and $CH_2OH$. In some embodiments, V is selected from -COOH.

In some embodiments, the spacer unit comprises:

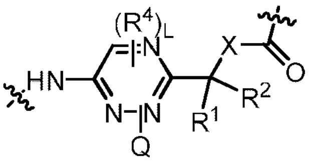

,

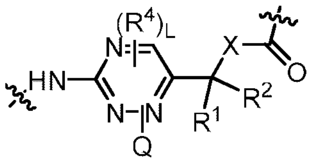

,

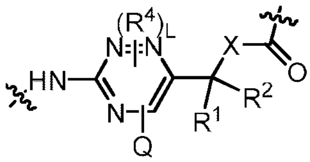

,

or

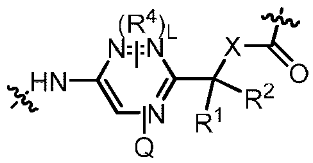

.

In another aspect, L-D in the antibody conjugate (ADC) of the present disclosure is a chemical moiety represented by the formula below:

-Str- (Pep)-Sp-D

wherein Str is a stretcher unit covalently linking to Ab,

Sp is a spacer unit, and

Pep is selected from the group consisting of amino acid units.

In another aspect, Str in the ADC is selected from a chemical moiety represented by the formula below:

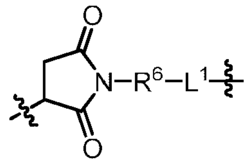

,

wherein $R^6$ is selected from the group consisting of -W-C(O)-, -C(O)-W-C(O)-, $(CH_2CH_2O)_{p1}C(O)$-, $(CH_2CH_2O)_{p1}CH_2C(O)$-, and $(CH_2CH_2O)_{p1}CH_2CH_2C(O)$-, wherein W is selected from the group consisting of $C_{1-8}$ alkylene, $C_{1-8}$ alkylene-cycloalkyl, and linear heteroalkylene with 1 to 8 atoms, and the heteroalkylene comprises 1 to 3 heteroatoms selected from the group consisting of N, O, and S, wherein the $C_{1-8}$ alkylene, $C_{1-8}$ alkylene-cycloalkyl, and linear heteroalkylene are each independently optionally further substituted with one or more substituents selected from the group consisting of halogen, hydroxy, cyano, amino, alkyl, chloroalkyl, alkoxy, and cycloalkyl;

$L^1$ is selected from the group consisting of $-NR^7(CH_2CH_2O)_{p1}CH_2CH_2C(O)$-, - $NR^7(CH_2CH_2O)_{Pl}CH_2C(O)$-, $-S(CH_2)_{p1}C(O)$-, $-(CH_2)_{p1}C(O)$-, and a chemical bond, preferably a chemical bond, wherein p1 is an integer from 1 to 20, and $R^7$ is selected from the group consisting of hydrogen, alkyl, haloalkyl, and hydroxyalkyl.

In some embodiments, the linker may comprise at least one polyethylene glycol (PEG) moiety. The PEG moiety may, e.g., comprise $-(PEG)_{p1}$-,

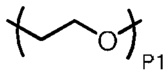

,

wherein p1 is an integer from 1 to 20, such as

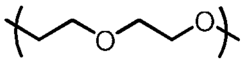

,

$(PEG)_2$;

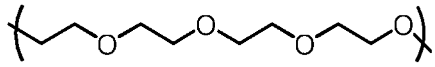

,

$(PEG)_4$;

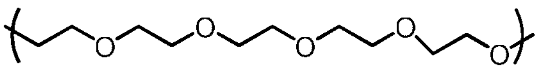

,

$(PEG)_5$.

In some embodiments, the spacer unit in the linker comprises $(PEG)_2$. In some embodiments, the ADC comprising a shorter spacer unit (e.g., $(PEG)_2$) shows a lower aggregation level and/or a higher drug loading relative to the ADC comprising a longer spacer unit (e.g., $(PEG)_8$) despite the shorter linker length.

In some embodiments, $R^7$ in the antibody-drug conjugate is selected from the group consisting of $C_{1-6}$ alkylene C(O)-, $-(CH_2-CH_2O)_2C(O)$-, $-(CH_2-CH_2O)_2CH_2C(O)$-, $-(CH_2-CH_2O)_2CH_2CH_2C(O)$-, $-(CH_2-CH_2O)_3C(O)$-, and $-(CH_2-CH_2O)_4C(O)$-.

In some embodiments, the linker L in the antibody-drug conjugate comprises: maleimide-$(PEG)_2$-Val-Cit, maleimide-$(PEG)_6$-Val-Cit, maleimide-$(PEG)_8$-Val-Cit, maleimide-$(PEG)_4$-$CH_2CH_2C(O)$-Val-lys, maleimide-$(CH_2)_5$-Val-Cit, maleimide-$(CH_2)_5$-Val-lys, maleimide-$(CH_2)_5$-Gly-Gly-Phe-Gly, maleimide-$(PEG)_2$-Ala-Ala-Asn, maleimide-$(PEG)_6$-Ala-Ala-Asn, maleimide-(PEG)s-Ala-Ala-Asn, maleimide-$(PEG)_4$-triazole-$(PEG)_3$-sulfonamide, maleimide-$(PEG)_2$-$CH_2CH_2C(O)$-Val-lys, maleimide-$(PEG)_4$-triazole-$(PEG)_3$-sulfonamide, or Mal-$(PEG)_4$-triazole-$(PEG)_3$-disulfide.

In another aspect, Str in the antibody-drug conjugate provided by some embodiments is selected from a chemical moiety represented by the formula below:

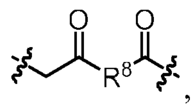

wherein $R^8$ is selected from the group consisting of $C_{1-10}$ alkylene, $C_{2-10}$ alkenyl, ($C_{1-10}$ alkylene)O-, $N(R^d)$-($C_{2-6}$ alkylene)-$N(R^d)$, and $N(R^d)$-($C_{2-6}$ alkylene); each $R^d$ is independently H or $C_{1-6}$ alkyl.

In some embodiments, the antibody-drug conjugate is represented by the formulas below:

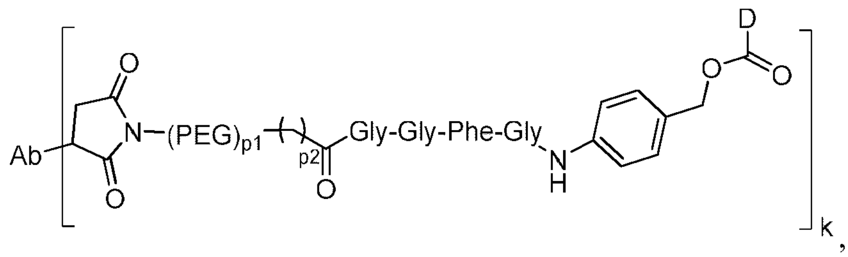

wherein k is selected from the group consisting of 1 to 10 and can be an integer or a decimal; p1 is selected from the group consisting of 2, 4, 6, and 8; p2 is selected from the group consisting of 0, 1, and 2;

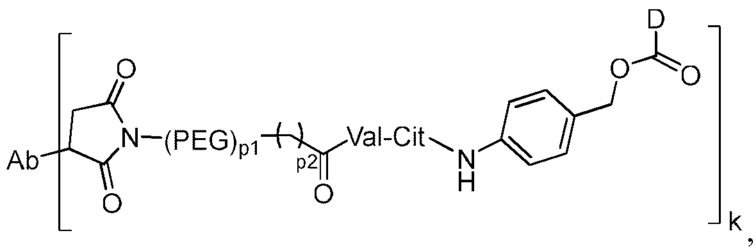

wherein k is selected from the group consisting of 1 to 10 and can be an integer or a decimal; p1 is selected from the group consisting of 2, 4, 6, and 8; p2 is selected from the group consisting of 0, 1, and 2;

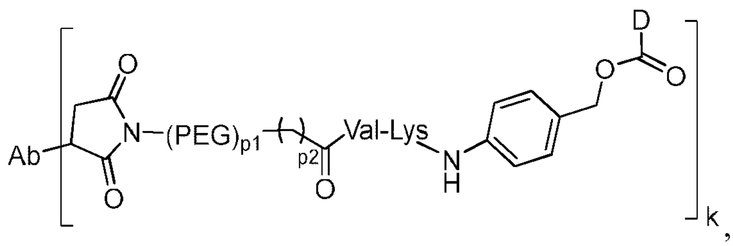

wherein k is selected from the group consisting of 1 to 10 and can be an integer or a decimal; p1 is selected from the group consisting of 2, 4, 6, and 8; p2 is selected from the group consisting of 0, 1, and 2;

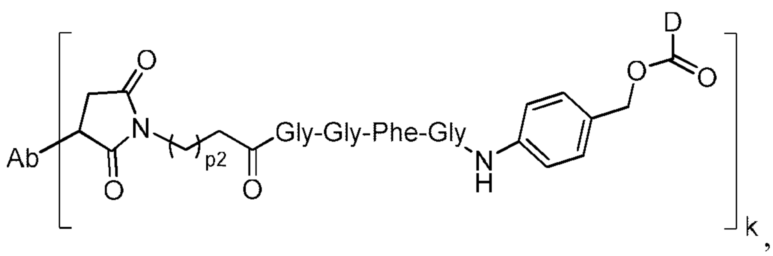

wherein k is selected from the group consisting of 1 to 10 and can be an integer or a decimal; p2 is selected from the group consisting of integers from 1 to 6;

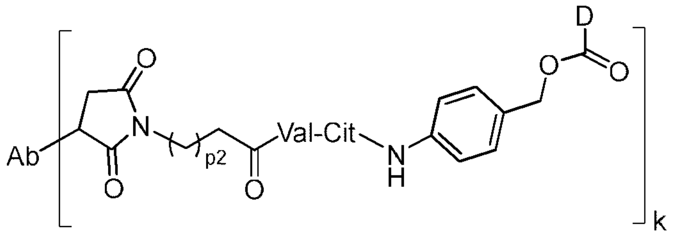,

wherein k is selected from the group consisting of 1 to 10 and can be an integer or a decimal; p2 is selected from the group consisting of integers from 1 to 6;

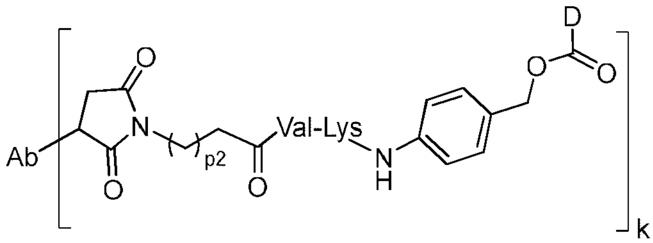,

wherein k is selected from the group consisting of 1 to 10 and can be an integer or a decimal; p2 is selected from the group consisting of integers from 1 to 6.

Further, the antibody-drug conjugate (ADC) of the present disclosure is selected from the group consisting of:

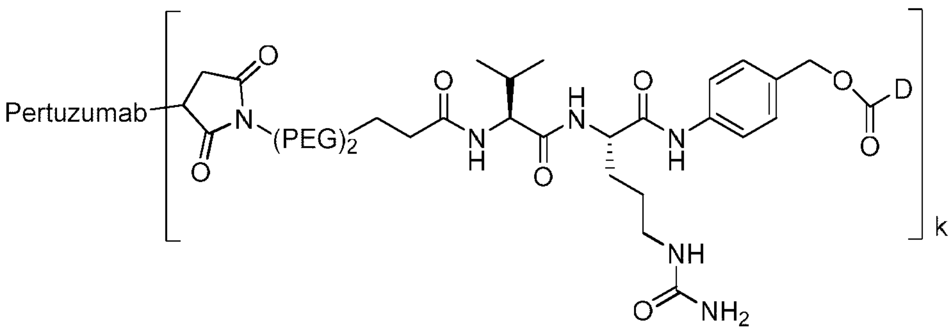

,

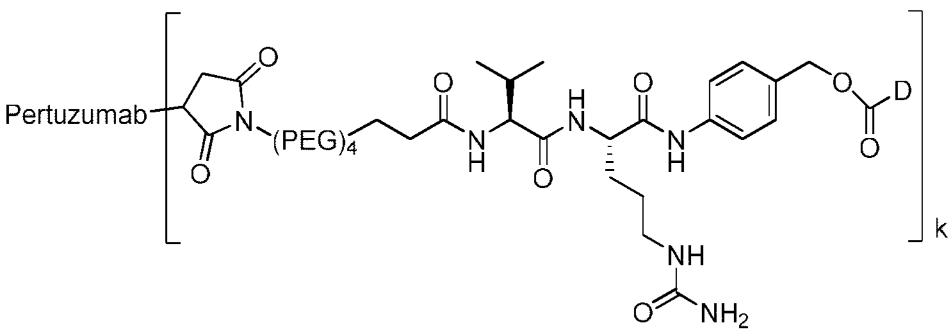

,

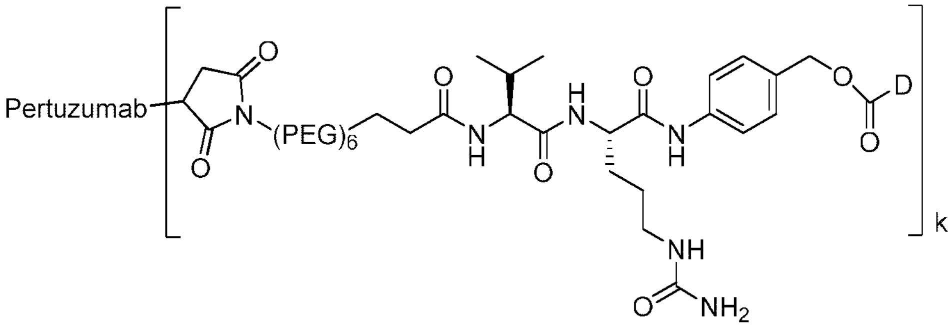

,

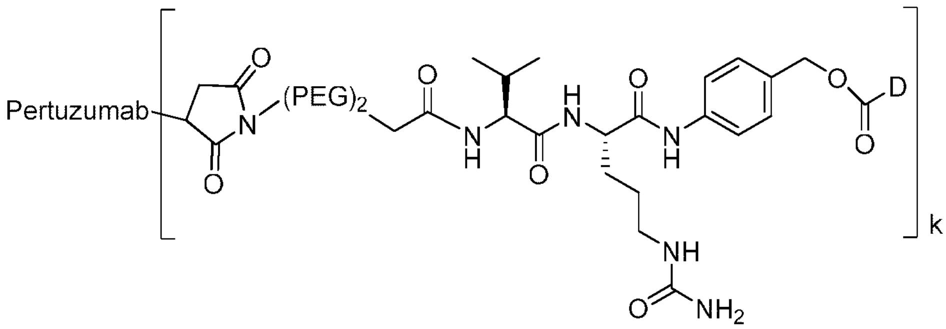

,

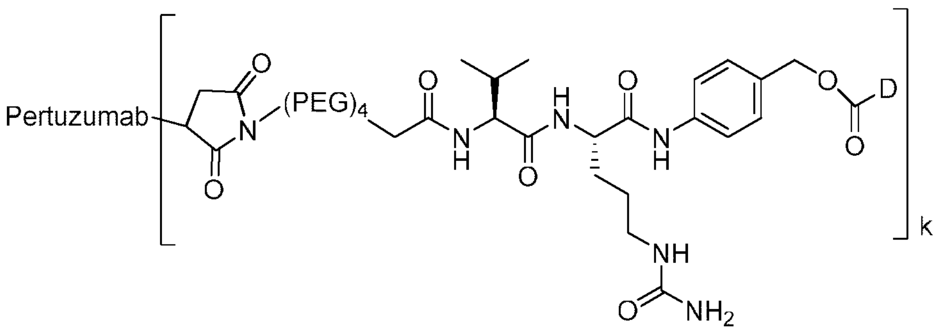

,

and

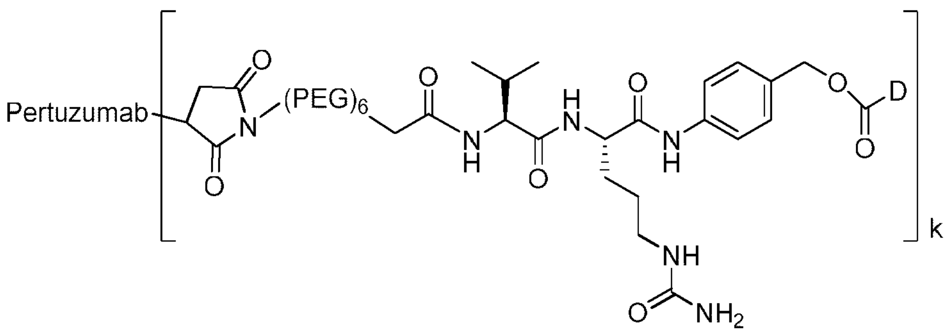

,

wherein k is selected from the group consisting of 1 to 10 and can be an integer or a decimal.

The sequences of the heavy chain variable region and the light chain variable region of the HER2 antibody or the antigen-binding fragment thereof to which the domain II of HER2 described above binds are set forth below:

Light chain variable region

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIK

SEQ ID NO: 1

Heavy chain variable region

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSS

SEQ ID NO: 2.

The following is the sequence of pertuzumab:

Light chain

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 3

Heavy chain

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 4.

The following is the sequence of trastuzumab:

Light chain

DIQMTQSPSSLSASVGDRVTITCRASQDVNTAVAWYQQKPGKAPKLLIYSASFLY
SGVPSRFSGSRSGTDFTLTISSLQPEDFATYYCQQHYTTPPTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 5

Heavy chain

EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 6.

In some embodiments, k in the antibody conjugate of the present disclosure is selected from 2.0 to 2.5, including 2.0, 2.1, 2.2, 2.3, 2.4, 2.5, or a value between any two values. In some other embodiments, k in the antibody conjugate of the present disclosure is selected from 2.5 to 3.5, including 2.5, 2.6, 2.7, 2.8, 2.9, 3.0, 3.1, 3.2, 3.3, 3.4, 3.6, 3.7, 3.8, 3.9, 4.0, or a value between any two values.

In some other embodiments, k in the antibody conjugate of the present disclosure is selected from 3.5 to 5.0, including 3.5, 3.6, 3.7, 3.8, 3.9, 4.0, 4.1, 4.2, 4.3, 4.4, 4.5, 4.6, 4.7, 4.8, 4.9, 5.0, or a value between any two values.

In another aspect, the present disclosure also provides an isotopically substituted form of the antibody conjugate described above. In some embodiments, the isotopically substituted form is obtained by a substitution with a deuterium atom.

In another aspect, the present disclosure also provides a pharmaceutical composition comprising a therapeutically effective amount of the antibody-drug conjugate or the isotopically substituted form thereof described above and a pharmaceutically acceptable carrier, diluent, or excipient.

In some embodiments, a unit dose of the pharmaceutical composition is 0.001 mg-1000 mg.

In certain embodiments, the pharmaceutical composition comprises 0.01%-99.99% of the antibody-drug conjugate or the isotopically substituted form thereof described above based on the total weight of the composition. In certain embodiments, the pharmaceutical composition comprises 0.1%-99.9% of the antibody-drug conjugate or the isotopically substituted form thereof described above. In certain embodiments, the pharmaceutical composition comprises 0.5%-99.5% of the antibody-drug conjugate or the isotopically substituted form thereof described above. In certain embodiments, the pharmaceutical composition comprises 1%-99% of the antibody-drug conjugate or the isotopically substituted form thereof described above. In certain embodiments, the pharmaceutical composition comprises 2%-98% of the antibody-drug conjugate or the isotopically substituted form thereof described above.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of a pharmaceutically acceptable excipient. In certain embodiments, the pharmaceutical composition comprises 0.5%-99.5% of a pharmaceutically acceptable excipient. In certain embodiments, the pharmaceutical composition comprises 1%-99% of a pharmaceutically acceptable excipient. In certain embodiments, the pharmaceutical composition comprises 2%-98% of a pharmaceutically acceptable excipient.

The present disclosure also provides use of the antibody-drug conjugate or the pharmaceutical composition described above in preparing a medicament for treating or preventing a tumor. In some embodiments, the tumor is cancer associated with expression of a domain II of HER2.

The present disclosure also provides use of the antibody-drug conjugate or the pharmaceutical composition described above in preparing a medicament for treating and/or preventing cancer. In some embodiments, the cancer is selected from the group consisting of breast cancer, ovarian cancer, cervical cancer, uterine cancer, prostate cancer, kidney cancer, urinary tract cancer, bladder cancer, liver cancer, gastric cancer, endometrial cancer, salivary gland carcinoma, esophageal cancer, melanoma, neuroglioma, neuroblastoma, sarcoma, lung cancer, colon cancer, rectal cancer, colorectal cancer, leukemia, bone cancer, skin cancer, thyroid cancer, pancreatic cancer, and lymphoma.

The present disclosure also provides a method for treating or preventing cancer associated with expression of a domain II of HER2 in a patient, which comprises administering to the patient a therapeutically effective amount of the antibody-drug conjugate or the isotopically substituted form thereof described above or the pharmaceutical composition described above.

The present disclosure also provides the antibody-drug conjugate or the isotopically substituted form thereof described above or the pharmaceutical composition described above for use in treating or preventing cancer associated with expression of a domain II of HER2.

The active compound may be formulated into a form suitable for administration by any suitable route, preferably in a form of a unit dose, or in a form of a single dose that can be self-administered by a patient. The unit dose of the compound or composition of the present invention may be expressed in the form of a tablet, a capsule, a cachet, a vial, a powder, a granule, a lozenge, a suppository, a regenerating powder, or a liquid formulation.

## Detailed Description of the Invention

Unless defined otherwise, all technical and scientific terms used herein have the same meaning as commonly understood by those of ordinary skill in the art to which the present disclosure belongs. Although any methods and materials similar or equivalent to those of the present disclosure can also be used to implement or test the present disclosure, preferred methods and materials are described herein. In describing and claiming the present disclosure, the following terms are used in accordance with the definitions below. When a trade name is used in the present disclosure, the applicant intends to include the formulation of the commercial product under the trade name, and the non-patent drug and active drug component of the commercial product under the trade name.

Unless otherwise stated, the terms used in the specification and claims have the following meanings.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure. The compounds of the present disclosure containing asymmetric carbon atoms can be isolated in optically active pure form or in racemic form. The optically active pure form can be isolated from a racemic mixture or synthesized using chiral starting materials or chiral reagents.

Optically active (R)- and (S)-enantiomers and D- and L-isomers can be prepared by chiral synthesis, chiral reagents or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, followed by resolution of diastereomers by conventional methods known in the art, and the pure enantiomers are obtained by recovery. In addition, separation of enantiomers and diastereomers is generally accomplished by chromatography using a chiral stationary phase, optionally in combination with chemical derivatization (e.g., carbamate formation from amines).

In the chemical structure of the compound of the present disclosure, a bond "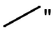" represents an unspecified configuration; that is, if chiral isomers exist in the chemical structure, the bond "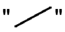" may be "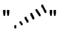" or "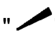", or contains both the configurations of "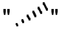" and "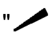". The bond represents an unspecified configuration, including the cis (E) or trans (Z) configuration. Or "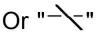" described herein refers to a double bond, and a structure featuring bonding via this bond may be a "cis isomer", a "trans isomer" or "a mixture of cis and trans isomers in any ratio". For example, formula E represents formula E-1, formula E-2, or a mixture of both in any ratio:

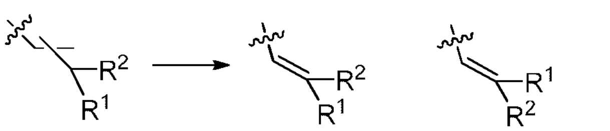

Formula E Formula E-1 Formula E-2.

In the chemical structure of the compound described herein, a bond "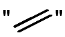" is not specified with a configuration; that is, it may be in a Z configuration or an E configuration, or contains both configurations.

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, proton tautomers (also known as proton transfer tautomers) include interconversion via proton migration, such as keto-enol and imine-enamine, lactam-lactim isomerization. An example of a lactam-lactim equilibrium is present between A and B as shown

below.

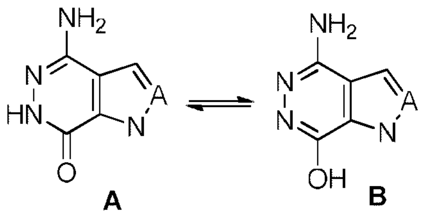

A B

All compounds in the present disclosure can be drawn as form A or form B. All tautomeric forms are within the scope of the present disclosure. The nomenclature of the compounds does not exclude any tautomers.

The present disclosure further includes isotopically-labeled compounds that are identical to those recited herein but have one or more atoms replaced by an atom having an atomic mass or mass number different from the atomic mass or mass number usually found in nature. Examples of isotopes that can be incorporated into the compound of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}0$, $^{31}P$, $^{32}P$, $^{35}S$, $^{18}F$, $^{123}I$, $^{125}I$ and $^{36}Cl$.

Unless otherwise specified, when a position is specifically assigned deuterium (D), the position should be construed as deuterium with an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further includes various deuterated forms of the compound of formula (I). Each available hydrogen atom connected to a carbon atom may be independently replaced by a deuterium atom. Those skilled in the art are able to synthesize the deuterated forms of the compound of formula (I) according to the relevant literature. Commercially available deuterated starting materials can be used in preparing the deuterated forms of the compound of formula (I), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Optionally" or "optional" means that the event or circumstance subsequently described may, but does not necessarily, occur and that the description includes instances where the event or circumstance occurs or does not occur. For example, "$C_{1-6}$ alkyl that is optionally substituted with a halogen or cyano" means that the halogen or cyano may, but does not necessarily, exist, and the description includes the instance where alkyl is substituted with a halogen or cyano and the instance where alkyl is not substituted with a halogen or cyano. "Pharmaceutical composition" refers to a mixture containing one or more of the compounds or the physiologically/pharmaceutically acceptable salts or prodrugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

"Pharmaceutically acceptable excipient" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent or emulsifier that has been approved by the U.S. Food and Drug Administration as acceptable for use in humans or livestock animals.

"Effective amount" or "therapeutically effective amount" described herein includes an amount sufficient to ameliorate or prevent a symptom or disorder of a medical disorder. An effective amount also refers to an amount sufficient to allow or facilitate diagnosis. The effective amount for a particular patient or veterinary subject may vary with factors such as the disorder to be treated, the general health of the patient, the method and route and dosage of administration, and the severity of side effects. An effective amount may be the maximum dose or administration regimen to avoid significant side effects or toxic effects.

The term "drug" refers to a cytotoxic drug or an immunomodulator. The cytotoxic drug may have a chemical molecule within the tumor cell that is strong enough to disrupt its normal growth. The cytotoxic drug can kill tumor cells in principle at a sufficiently high concentration; however, due to lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant or animal origin, radioisotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), toxic drugs, chemotherapeutic drugs, antibiotics and nucleolytic enzymes. The immunomodulator is an inhibitor of immune checkpoint molecules.

The term "linker", "linker unit" or "linker fragment" refers to a chemical structural fragment or bond, which is linked to a ligand at one end and linked to a drug at the other end, and also may be linked to other linkers and then

linked to the drug.

The linker may comprise one or more linker components. Exemplary linker components include 6-maleimidocaproyl (MC), maleimidopropionyl (MP), valine-citrulline (Val-Cit or vc), alanine-phenylalanine (ala-phe), *p*-aminobenzyloxycarbonyl (PAB), and those derived from coupling to a linker reagent: *N*-succinimidyl 4-(2-pyridylthio)pentanoate (SPP), *N*-succinimidyl 4-(*N*-maleimidomethyl)cyclohexane-1 carboxylate (SMCC, also referred to herein as MCC), and *N*-succinimidyl(4-iodo-acetyl)aminobenzoate (SIAB). The linker can include a stretcher unit, a spacer unit, an amino acid unit and an extension unit. The linker may be synthesized using methods known in the art, such as those described in US2005-0238649A1. The linker may be a "cleavable linker" favoring the release of drugs in cells. For example, acid-labile linkers (e.g., hydrazones), proteasesensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

The term "stretcher unit" refers to a chemical structural fragment that covalently links to an antibody through carbon atoms at one end and is linked to an amino acid unit, a disulfide moiety, a sulfonamide moiety or a non-peptide chemical moiety at the other end. The term "spacer unit" is a bifunctional compound structural fragment that can be used to couple an amino acid unit to a cytotoxic drug to form an antibody-drug conjugate, in such a way that the cytotoxic drug is selectively linked to the amino acid unit.

The term "amino acid" refers to an organic compound that contains amino and carboxyl in the molecular structure and in which both amino and carboxyl are directly linked to a -CH- structure. The general formula is $H_2NCHRCOOH$, where R is H, substituted or unsubstituted alkyl, and the like. Amino acids are classified as α, β, γ, δ, ε...-amino acids according to the position of the carbon atom to which the amino is linked in the carboxylic acid. In the biological field, the amino acids that constitute native proteins have their specific structural characteristics; that is, their amino groups are attached directly to the α-carbon atoms, namely α-amino acids, including Gly (Glycine), Ala (Alanine), Val (Valine), Leu (Leucine), Ile (Isoleucine), Phe (Phenylalanine), Trp (Tryptophan), Tyr (Tyrosine), Asp (Aspartic acid), His (Histidine), Asn (Asparagine), Glu (Glutamic acid), Lys (Lysine), Gln (Glutamine), Met (Methionine), Arg (Arginine), Ser (Serine), Thr (Threonine), Cys (Cysteine), Pro (Proline), etc. Non-natural amino acids are, for example, citrulline. As is well known to those skilled in the art, non-natural amino acids do not constitute natural proteins and are therefore not involved in the synthesis of antibodies in the present disclosure. The three-letter and single-letter codes for amino acids used in the present disclosure are as described in J. Biol. Chem, 243, p3558 (1968).

| For short | Abbreviation | Name | Structure |
|---|---|---|---|
| G | Gly | Glycine | 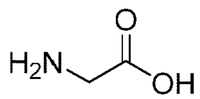 |
| A | Ala | Alanine | 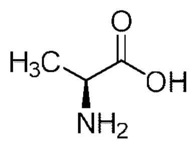 |
| V | Val | Valine | 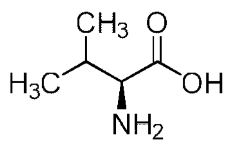 |
| L | Leu | Leucine | 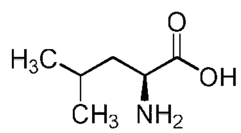 |
| I | Ile | Isoleucine | 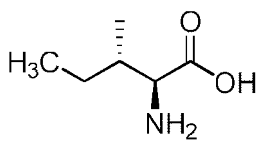 |

(continued)

| For short | Abbreviation | Name | Structure |
|---|---|---|---|
| F | Phe | Phenylalanine | 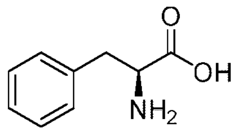 |
| W | Trp | Tryptophan | 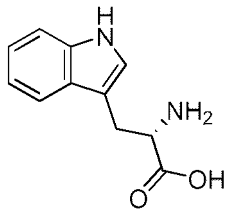 |
| Y | Tyr | Tyrosine | 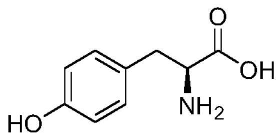 |
| D | Asp | Aspartic acid | 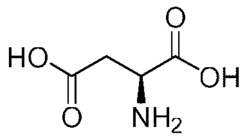 |
| H | His | Histidine | 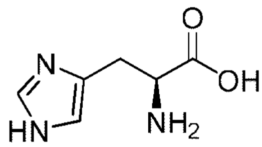 |
| N | Asn | Asparagine | 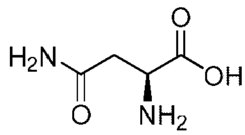 |
| E | Glu | Glutamic acid | 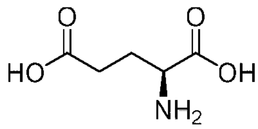 |
| K | Lys | Lysine | 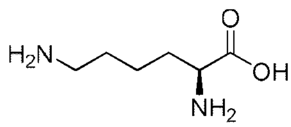 |
| Q | Gln | Glutamine | 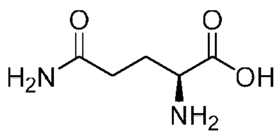 |
| C | Cit | Citrulline | 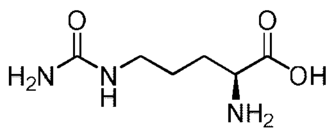 |

The spacer unit in the present disclosure is PAB that has a structure represented by a *p*-aminobenzyloxycarbonyl

fragment, has a structure represented by formula (VI), and is linked to D,

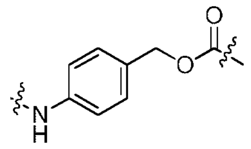

(VI).

Linker components include, but are not limited to:

MC = 6-maleimidocaproyl, whose structure is shown below:

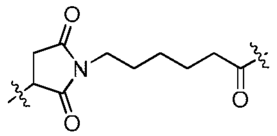

Val-Cit or "vc" = valine-citrulline (an exemplary dipeptide in a protease cleavable linker) citrulline = 2-amino-5-ureidopentanoic acid
Me-Val-Cit = N-methyl-valine-citrulline (wherein the linker peptide bond has been modified to prevent it from being cleaved by cathepsin B)
$MC(PEG)_6$-OH = maleimidocaproyl-polyethylene glycol (attachable to antibody cysteine)
SPP = *N*-succinimidyl 4-(2-pyridylthio)valerate
SPDP = *N*-succinimidyl 3-(2-pyridyldithio)propionate
SMCC = succinimidyl-4-(*N*-maleimidomethyl)cyclohexane-1-carboxylate
IT = iminothiolane
PBS = phosphate-buffered saline.

The term "antibody-drug conjugate" means that a ligand is linked to a biologically active drug by a stable linker unit. In the present disclosure, "antibody-drug conjugate" (ADC) means that a monoclonal antibody or an antibody fragment is linked to a biologically active toxic drug by a stable linker unit.

The term "drug loading" may be expressed as the ratio of the amount of drug to the amount of antibody, i.e., the average number of drugs conjugated per antibody in the ADC. The drug loading may range from 1 to 20, preferably from 1 to 10 cytotoxic drugs (D) attached per antibody (Ab). In embodiments of the present disclosure, the drug loading is represented as k, and may illustratively be 1, 2, 3, 4, 5, 6, 7, 8, 9, 10, 11, 12, 13, 14, 15, 16, 17, 18, 19, 20, or an average of any two values; preferably an average of 1 to 10, and more preferably an average of 1 to 8, or 2 to 8, or 2 to 7, or 3 to 8, or 3 to 7, or 3 to 6, or 4 to 7, or 4 to 6, or 4 to 5. The average number of drugs per ADC molecule after coupling reactions can be characterized by conventional methods such as UV/visible spectroscopy, mass spectrometry, ELISA assays, monoclonal antibody molecule size variant assay (CE-SDS) and HPLC.

The monoclonal antibody molecular size variant assay (CE-SDS) of the present disclosure may be used for quantitatively determining the purity of a recombinant monoclonal antibody product by adopting capillary electrophoresis-sodium dodecyl sulfate (CE-SDS) ultraviolet assay based on the molecular weight under reduced and nonreduced conditions and according to a capillary electrophoresis method (Chinese Pharmacopoeia 0542, 2015 Edition).

The loading of the antibody-drug conjugate can be controlled by the following non-limiting methods, including:

(1) controlling a molar ratio of a linking reagent to a monoclonal antibody,
(2) controlling reaction time and temperature, and
(3) selecting different reaction reagents.

The term "antibody" refers to an immunoglobulin, which is of a tetrapeptide chain structure formed by connection between two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid

composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain. In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxyl-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3.

The antibody of the present disclosure includes a murine antibody, a chimeric antibody, a humanized antibody and a fully human-derived antibody, preferably a humanized antibody and a fully human-derived antibody.

The term "murine antibody" used herein refers to an antibody prepared from mice according to the knowledge and skill in the art. During the preparation, a test subject is injected with a specific antigen, and then hybridomas expressing antibodies with desired sequences or functional properties are isolated.

The term "chimeric antibody" refers to an antibody obtained by fusing a variable region of a murine antibody to a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, connecting the mouse variable region gene and the human constant region gene into a chimeric gene, inserting the chimeric gene into an expression vector, and finally expressing chimeric antibody molecules in a eukaryotic system or prokaryotic system.

The term "humanized antibody", also known as a CDR-grafted antibody, refers to an antibody produced by grafting murine CDR sequences into a human antibody variable region framework, i.e., a different type of human germline antibody framework sequence. Such an antibody can overcome the heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human germline sequence database (available at the Internet address www.mrccpe.com.ac.uk/vbase), as well as in Kabat, E. A. et al., 1991 Sequences of Proteins of Immunological Interest, 5th edition. To avoid the decrease in activity caused by the decrease in immunogenicity, the FR sequence in the human antibody variable region can be subjected to minimum reverse mutation or back mutation to maintain activity. The humanized antibody of the present disclosure also includes the humanized antibody formed after further affinity maturation on the CDRs by phage display. Literature further describing methods used in humanization of accessible mouse antibodies includes, for example, Queen et al., Proc. Natl. Acad. Sci. USA, 88, 2869, 1991, and literature describing humanization using the method provided by Winter and coworkers thereof includes Jones et al., Nature, 321, 522 (1986); Riechmann et al., Nature, 332, 323-327 (1988), Verhoeyen et al, Science, 239, 1534 (1988).

The term "fully human-derived antibody", "fully human antibody" or "completely human-derived antibody", also known as "fully human-derived monoclonal antibody", may have both humanized variable regions and constant regions, thus eliminating immunogenicity and toxic and side effects. The development of monoclonal antibodies has four stages, namely murine monoclonal antibodies, chimeric monoclonal antibodies, humanized monoclonal antibodies and fully human-derived monoclonal antibodies. The antibody of the present disclosure is the fully human-derived monoclonal antibody. Major relevant technologies for the preparation of the fully human antibody include human hybridoma technology, EBV-transformed B-lymphocyte technology, phage display technology, transgenic mouse antibody preparation technology, single B-cell antibody preparation technology, and the like.

The term "antigen-binding fragment" refers to one or more fragments of an antibody that retain the ability to specifically bind to an antigen. It is shown that a fragment of a fulllength antibody can be used to perform the antigen-binding function of the antibody. The examples of the binding fragment included in the "antigen-binding fragment" include (i) Fab fragments, monovalent fragments consisting of VL, VH, CL and CH1 domains; (ii) $F(ab')_2$ fragments, bivalent fragments comprising two Fab fragments connected by disulfide bridges in the hinge regions; (iii) Fd fragments consisting of VH and CH1 domains; (iv) Fv fragments consisting of VH and VL domains of a single arm of an antibody; (v) single domains or dAb fragments (Ward et al., (1989) Nature 341: 544-546) consisting of VH domains; and (vi) isolated complementarity determining regions (CDRs) or (vii) combinations of two or more isolated CDRs which may optionally be linked by synthetic linkers. In addition, although the two domains of the Fv fragment, VL and VH, are encoded by separate genes, they can be linked by a synthetic linker by a recombination method, thus producing a single protein chain in which the VL and VH regions pair to form a monovalent molecule (referred to as single-chain Fv (scFv); see, e.g., Bird et al. (1988) Science 242: 423-426; and Huston et al. (1988) Proc. Natl. Acad. Sci. USA 85: 5879-5883). Such single-chain antibodies are also intended to be included in the term "antigen-binding fragment" of an antibody. Such antibody fragments are obtained by conventional techniques known to those skilled in the art, and screened for utility in the same manner as for intact antibodies. Antigen-binding moieties may be produced by a recombinant DNA technique or by enzyme catalysis or chemical cleavage of intact immunoglobulins. The antibody may be of different isotypes, e.g., an IgG (e.g., subtype IgG1, IgG2, IgG3 or IgG4), IgA1, IgA2, IgD, IgE or IgM antibody.

Fab is an antibody fragment having a molecular weight of about 50,000 and having antigen-binding activity, among fragments obtained by treating an IgG antibody molecule with a protease papain (cleaving the amino acid residue at position 224 of H chain), in which about half of the N-terminal side of the H chain and the entire L chain are joined together by disulfide bonds.

F(ab')2 is an antibody fragment having a molecular weight of about 100,000 and having antigen-binding activity and comprising two Fab regions linked at the hinge position, which is obtained by digesting a portion below two disulfide bonds in the IgG hinge region with the enzyme pepsin.

Fab' is an antibody fragment having a molecular weight of about 50,000 and having an antigen-binding activity, which is obtained by cleaving the disulfide bond in the hinge region of the F(ab')2 described above.

In addition, the Fab' can be produced by inserting DNA encoding the Fab' fragment of the antibody into a prokaryotic expression vector or a eukaryotic expression vector and introducing the vector into a prokaryote or a eukaryote to express the Fab'.

The term "single-chain antibody", "single-chain Fv" or "scfv" refers to a molecule comprising an antibody heavy chain variable domain (or region; VH) and an antibody light chain variable domain (or region; VL) linked by a linker. Such scFv molecules may have a general structure: $NH_2$-VL-linker-VH-COOH or $NH_2$-VH-linker-VL-COOH. Suitable linkers in the prior art consist of repeated GGGGS amino acid sequences or variants thereof, for example, 1-4 repeated variants (Holliger et al. (1993), Proc. Natl. Acad. Sci. USA 90: 6444-6448). Other linkers that can be used in the present disclosure are described in Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol.

The term "CDR" refers to one of the 6 hypervariable regions within the variable domain of an antibody which primarily contribute to antigen binding. One of the most common definitions for the 6 CDRs is provided in Kabat E.A. et al., (1991) Sequences of proteins of immunological interest. NIH Publication 91-3242. As used herein, the Kabat definition of CDRs may be only applied to CDR1, CDR2 and CDR3 of the light chain variable domain (CDR L1, CDR L2, CDR L3 or L1, L2, L3), and CDR2 and CDR3 of the heavy chain variable domain (CDR H2, CDR H3 or H2, H3). Generally, there are three CDRs (HCDR1, HCDR2 and HCDR3) in each heavy chain variable region and three CDRs (LCDR1, LCDR2 and LCDR3) in each light chain variable region. The amino acid sequence boundaries of the CDRs can be determined using any of a variety of well-known schemes, including "Kabat" numbering scheme (see Kabat et al. (1991), "Sequences of Proteins of Immunological Interest", 5th edition, Public Health Service, National Institutes of Health, Bethesda, MD), "Chothia" numbering scheme (see Al-Lazikani et al. (1997), JMB 273: 927-948) and ImMunoGenTics (IMGT) numbering scheme (see Lefranc M.P., Immunologist, 7, 132-136(1999); Lefranc, M.P. et al., Dev. Comp. Immunol., 27, 55-77(2003)), and the like. For example, for the classical format, according to the Kabat scheme, the CDR amino acid residues in the heavy chain variable domain (VH) are numbered 31-35 (HCDR1), 50-65 (HCDR2) and 95-102 (HCDR3); the CDR amino acid residues in the light chain variable domain (VL) are numbered 24-34 (LCDR1), 50-56 (LCDR2) and 89-97 (LCDR3). According to the Chothia scheme, the CDR amino acids in VH are numbered 26-32 (HCDR1), 52-56 (HCDR2) and 95-102 (HCDR3); and the amino acid residues in VL are numbered 26-32 (LCDR1), 50-52 (LCDR2) and 91-96 (LCDR3). According to the combination of CDR definitions provided in the Kabat scheme and the Chothia scheme, the CDR is composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. According to the IMGT scheme, the CDR amino acid residues in VH are roughly numbered 26-35 (CDR1), 51-57 (CDR2) and 93-102 (CDR3), and the CDR amino acid residues in VL are roughly numbered 27-32 (CDR1), 50-52 (CDR2) and 89-97 (CDR3).

According to the IMGT scheme, the CDRs of the antibody can be determined using the program IMGT/DomainGap Align.

The term "antibody framework" refers to a portion of a variable domain VL or VH, which serves as a framework for the antigen-binding loops (CDRs) of the variable domain. It is essentially a variable domain without CDRs.

The term "epitope" or "antigenic determinant" refers to a site on an antigen to which an immunoglobulin or antibody specifically binds. Epitopes generally comprise at least 3, 4, 5, 6, 7, 8, 9, 10, 11, 12, 13, 14 or 15 contiguous or non-contiguous amino acids in a unique spatial conformation (see, e.g., Epitope Mapping Protocols in Methods in Molecular Biology, Vol. 66, G.E.Morris, Ed. (1996)).

The terms "specific binding", "selective binding", "selectively bind to" and "specifically bind to" refer to the binding of an antibody to an epitope on a predetermined antigen. Generally, the antibody binds with an affinity (KD) of less than about $10^{-7}$ M, e.g., less than about $10^{-8}$ M, $10^{-9}$ M, or $10^{-10}$ M or less.

The term "nucleic acid molecule" refers to a DNA molecule and an RNA molecule. The nucleic acid molecule

may be single-stranded or double-stranded, and is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The term "vector" refers to a nucleic acid molecule capable of transporting another nucleic acid to which it has been linked. In one embodiment, the vector is a "plasmid" that refers to a circular double-stranded DNA loop into which additional DNA segments may be ligated. In another embodiment, the vector is a viral vector, wherein additional DNA segments may be ligated into the viral genome. The vectors disclosed herein are capable of autonomously replicating in a host cell into which they are introduced (e.g., bacterial vectors having a bacterial origin of replication and episomal mammalian vectors) or capable of integrating into the genome of a host cell after being introduced into the host cell and thus replicating with the host genome (e.g., non-episomal mammalian vectors).

Methods for producing and purifying antibodies and antigen-binding fragments are well known in the prior art, for example, those described in chapters 5-8 and 15 of "Antibodies: A Laboratory Manual", Cold Spring Harbor. Likewise, antigen-binding fragments can be prepared by conventional methods. The antibody or antigen-binding fragment described in the present invention is genetically engineered to contain one or more additional human-derived FRs in the non-human-derived CDRs. Human FR germline sequences can be obtained at the website http://imgt.cines.fr of ImMunoGeneTics (IMGT) or from the immunoglobulin journal, 2001ISBN012441351, by comparing the IMGT human antibody variable region germline gene database with the MOE software.

The term "host cell" refers to a cell into which an expression vector has been introduced. Host cells may include bacterial, microbial, plant or animal cells. Bacteria susceptible to transformation include members of the *Enterobacteriaceae* family, such as strains of *Escherichia coli* or *Salmonella;* members of the *Bacillaceae* family, such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conservative N-terminal site of the Fc region. Positive clones are expanded in a serum-free medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting product needs to be immediately frozen, e.g., at -70 °C, or lyophilized.

The amino acid sequence "identity" refers to the percentage of amino acid residues shared by a first sequence and a second sequence, wherein in aligning the amino acid sequences and when necessary, gaps are introduced to achieve maximum percent sequence identity, and any conservative substitution is not considered as part of the sequence identity. For the purpose of determining percent amino acid sequence identity, alignments can be achieved in a variety of ways that are within the scope of skills in the art, for example, using publicly available computer software such as BLAST, BLAST-2, ALIGN, ALIGN-2 or Megalign (DNASTAR) software. Those skilled in the art can determine parameters suitable for measuring alignment, including any algorithms required to achieve maximum alignment of the full length of the aligned sequences.

The term "peptide" refers to a compound fragment between an amino acid and a protein. The compound fragment is formed by connecting 2 or more amino acid molecules by peptide bonds, and is a structural and functional fragment of the protein, such as hormones and enzymes, which are essentially peptides.

The term "sugar" refers to biomacromolecules consisting of C, H and O elements. They can be classified into monosaccharides, disaccharides, polysaccharides and the like.

The term "fluorescent probe" refers to a class of fluorescent molecules that have characteristic fluorescence in the ultraviolet-visible-near infrared region and whose fluorescent properties (excitation and emission wavelengths, intensity, lifetime, polarization, etc.) can sensitively change as the properties of the environment they are in, such as polarity, refractive index and viscosity, change. These fluorescent molecules noncovalently interact with nucleic acids (DNA or RNA), proteins or other macromolecular structures to change one or more fluorescent properties, and therefore can be used to study the properties and behavior of macromolecular substances.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, *n*-propyl, isopropyl, *n*-butyl, isobutyl, *tert*-butyl, *n*-hexyl, 1-ethyl-2-methylpropyl, and various branched isomers thereof and the like. More preferred is a lower alkyl having 1 to 6 carbon atoms, and non-limiting examples include methyl, ethyl, *n*-propyl,

isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, and the like. The alkyl may be substituted or unsubstituted, and when it is substituted, the substituent may be substituted at any accessible connection site, and the substituent is preferably one or more groups independently selected from the group consisting of alkyl, alkoxy, halogen, amino, cyano, nitro, hydroxy, hydroxyalkyl, cycloalkyl, heterocyclyl, aryl and heteroaryl.

The term "heteroalkyl" refers to alkyl containing one or more heteroatoms selected from the group consisting of N, O and S, wherein the alkyl is as defined above.

The term "monovalent group" refers to an atom or group obtained by "formally" removing a monovalent atom or group from a compound. The term "ylene" refers to an atom or atomic group formed by "formally" removing two monovalent atoms or one divalent atoms from a compound. Exemplary "alkyl" refers to the moiety of an alkane molecule remaining after removal of 1 hydrogen atom, and includes a linear or branched monovalent group of 1 to 20 carbon atoms. Non-limiting examples of alkyl containing 1 to 6 carbon atoms include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, various branched isomers thereof, and the like.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene($-CH_2-$), 1,1-ethylidene($-CH(CH_3)-$), 1,2-ethylidene($-CH_2CH_2)-$, 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), etc. Alkylene may be substituted or unsubstituted, and when it is substituted, the substituent may be substituted at any accessible connection site, and the substituent is preferably independently optionally selected from one or more of halogen, hydroxy, cyano, amino, alkyl, chloroalkyl, alkoxy and cycloalkyl. Similarly, "alkenylene" is as defined above.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more groups independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spirocycloalkyl, fused cycloalkyl, and bridged cycloalkyl.

The term "heterocycloalkyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen and $S(O)_m$ (where m is an integer of 0 to 2), excluding a cyclic portion of -O-O-, -O-S-or -S-S-, and the remaining ring atoms are carbon atoms. The heterocycloalkyl preferably contains 3 to 12 ring atoms, of which 1 to 4 are heteroatoms; and more preferably contains 3 to 10 ring atoms. Non-limiting examples of monocyclic heterocycloalkyl include pyrrolidinyl, piperidinyl, piperazinyl, morpholinyl, thiomorpholinyl, homopiperazinyl, etc. Polycyclic heterocycloalkyl includes spiro heterocyclyl, fused heterocyclyl and bridged heterocycloalkyl.

The term "spiro heterocycloalkyl" refers to a 5- to 20-membered polycyclic heterocycloalkyl group in which monocyclic rings share one atom (referred to as the spiro atom), wherein one or more ring atoms are heteroatoms selected from the group consisting of nitrogen, oxygen and $S(O)_m$ (where m is an integer from 0 to 2), and the remaining ring atoms are carbon atoms. It may contain one or more double bonds, but none of the rings has a fully conjugated $\pi$-electron system. It is preferably 6- to 14-membered, and is more preferably 7- to 10-membered. According to the number of spiro atoms shared among the rings, the spiro heterocycloalkyl may be monospiro heterocycloalkyl, bispiro heterocycloalkyl or polyspiro heterocycloalkyl, preferably monospiro heterocycloalkyl and bispiro heterocycloalkyl, and more preferably 4-membered/4-membered, 4-membered/5-membered, 4-membered/6-membered, 5-membered/5-membered or 5-membered/6-membered monospiro heterocycloalkyl. Non-limiting examples of the spiro heterocycloalkyl include:

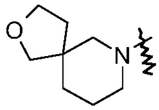 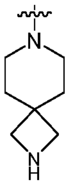 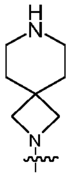 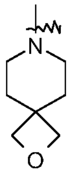 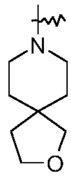 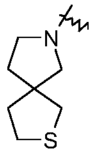 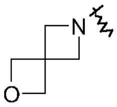 and 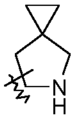 .

The term "fused heterocycloalkyl" refers to a 5- to 20-membered polycyclic heterocycloalkyl group in which each ring shares a pair of adjacent atoms with the other rings in the system, wherein one or more of the rings may contain one or more double bonds, but none of them has a fully conjugated $\pi$-electron system, wherein one or more of the ring atoms are heteroatoms selected from the group consisting of nitrogen, oxygen or $S(O)_m$ (where m is an integer of 0 to 2), and the remaining ring atoms are carbon atoms. It is preferably 6- to 14-membered, and is more preferably 7- to 10-membered. According to the number of the formed rings, the fused heterocycloalkyl may be bicyclic, tricyclic, tetracyclic or polycyclic, preferably bicyclic or tricyclic, and more preferably 5-membered/5-membered or 5-membered/6-membered bicyclic fused heterocycloalkyl. Non-limiting examples of the fused heterocycloalkyl include:

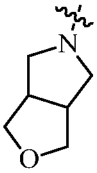 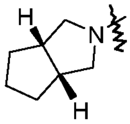 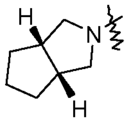 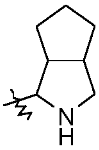 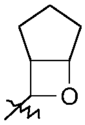 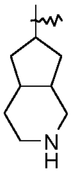 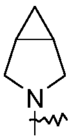 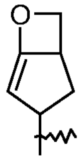 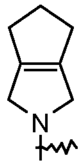

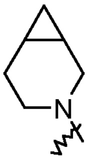 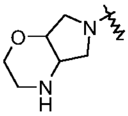 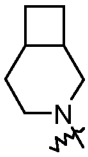 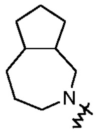 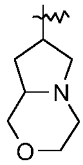 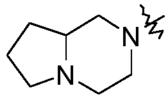

and

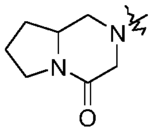 .

The term "bridged heterocycloalkyl" refers to a 5- to 14- membered polycyclic heterocycloalkyl group in which any two rings share two carbon atoms that are not directly attached to each other, wherein these rings may contain one or more double bonds, but none of them has a fully conjugated $\pi$-electron system, wherein one or more of the ring atoms are heteroatoms selected from the group consisting of nitrogen, oxygen and $S(O)_m$ (where m is an integer of 0 to 2), and the remaining ring atoms are carbon atoms. It is preferably 6- to 14-membered, and is more preferably 7- to 10-membered. According to the number of the formed rings, the bridged heterocycloalkyl may be bicyclic, tricyclic, tetracyclic or polycyclic, preferably bicyclic, tricyclic or tetracyclic, and more preferably bicyclic or tricyclic. Non-limiting examples of the bridged heterocycloalkyl include:

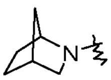 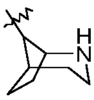 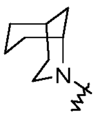 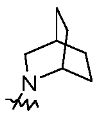

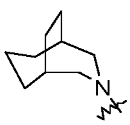 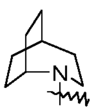 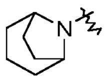 and 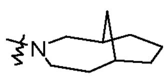 .

The heterocycloalkyl ring may be fused to an aryl, heteroaryl or cycloalkyl ring, wherein the ring attached to the parent structure is heterocycloalkyl; its non-limiting examples include, but are not limited to:

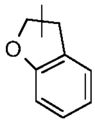 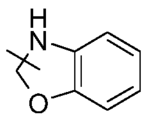 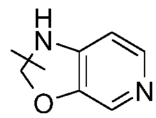, 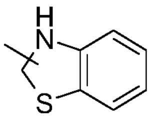,

etc.

The heterocycloalkyl may be optionally substituted or unsubstituted, and when it is substituted, the substituent is preferably one or more groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "aryl" refers to a 6- to 14-membered, preferably 6- to 10-membered, carbon monocyclic or fused polycyclic (i.e., rings sharing a pair of adjacent carbon atoms) group having a conjugated π-electron system, such as phenyl and naphthyl, preferably phenyl. The aryl ring may be fused to a heteroaryl, heterocycloalkyl or cycloalkyl ring, wherein the ring attached to the parent structure is the aryl ring; its non-limiting examples include, but are not limited to:

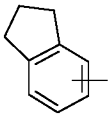 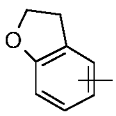 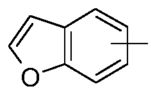 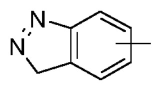 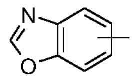 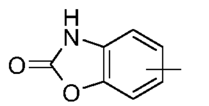

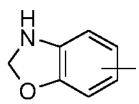 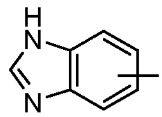 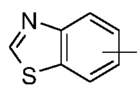 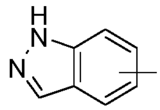 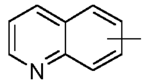 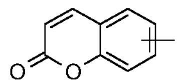

and

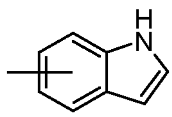.

The aryl may be substituted or unsubstituted, and when it is substituted, the substituent is preferably one or more groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered, more preferably 5- or 6-membered, e.g., furanyl, thienyl, pyridyl, pyrrolyl, N-alkylpyrrolyl, pyrimidinyl, pyrazinyl, imidazolyl and tetrazolyl. The heteroaryl ring may be fused to an aryl, heterocycloalkyl or cycloalkyl ring, wherein the ring attached to the parent structure is heteroaryl; its non-limiting examples include:

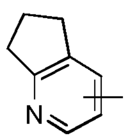 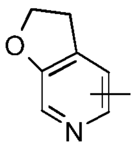 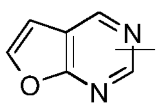 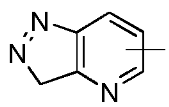 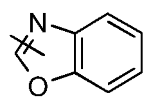 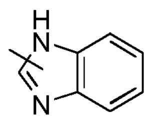

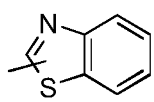 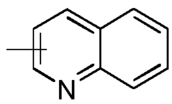 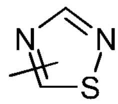

and

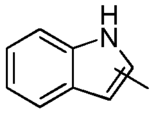

The heteroaryl may be optionally substituted or unsubstituted, and when it is substituted, the substituent is preferably one or more groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "aminoheterocycloalkyl" refers to heterocycloalkyl substituted with one or more amino groups, preferably one amino group, wherein the heterocycloalkyl is as defined above, and "amino group" means $-NH_2$. Representative examples of the present disclosure are as follows:

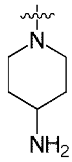,

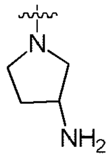.

The term "heterocycloalkylamino" refers to amino substituted with one or more heterocycloalkyl groups, preferably one heterocycloalkyl group, wherein the amino is as defined above, and the heterocycloalkyl is as defined above. Representative examples of the present disclosure are as follows:

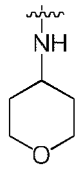.

The term "cycloalkylamino" refers to amino substituted with one or more cycloalkyl groups, preferably one cycloalkyl group, wherein the amino is as defined above, and the cycloalkyl is as defined above. Representative examples of the present disclosure are as follows:

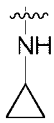

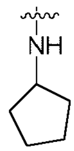.

The term "cycloalkylalkyl" refers to an alkyl group substituted with one or more cycloalkyl groups, preferably one cycloalkyl group, wherein the alkyl is as defined above, and the cycloalkyl is as defined above.

The term "haloalkyl" refers to alkyl substituted with one or more halogens, wherein the alkyl is as defined above.

The term "deuterated alkyl" refers to alkyl substituted with one or more deuterium atoms, wherein the alkyl is as defined above.

The term "hydroxy" refers to the -OH group.

The term "halogen" refers to fluorine, chlorine, bromine or iodine.

The term "amino" refers to $-NH_2$.

The term "nitro" refers to $-NO_2$.

In the chemical formula, the abbreviation "Me" refers to methyl.

"Optional" or "optionally" means that the event or circumstance subsequently described may, but does not necessarily, occur, and that the description includes instances where the event or circumstance occurs or does not occur. For example, "a heterocycloalkyl group optionally substituted with alkyl" means that alkyl may, but does not necessarily, exist and that the description includes instances where the heterocycloalkyl group is or is not substituted

with alkyl.

"Substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when an amino or hydroxy having free hydrogen is bound to a carbon atom having an unsaturated (e.g., olefinic) bond.

The term "pharmaceutically acceptable salt" refers to a salt of the antibody-drug conjugates of the present disclosure, or a salt of the compound described in the present disclosure. Such salts are safe and effective when used in the body of a mammal and possess the required biological activity. The antibody-drug conjugate of the present disclosure contains at least one amino group and thus can form salts with acids, and non-limiting examples of pharmaceutically acceptable salts include hydrochloride.

The term "solvate" refers to a pharmaceutical acceptable solvate formed by a ligand-drug conjugate compound of the present disclosure and one or more solvent molecules, and non-limiting examples of the solvent molecules include water, ethanol, acetonitrile, isopropanol, DMSO and ethyl acetate.

The term "drug carrier" for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals, vesicles and the like, as preferred examples. The aggregates have the capability of encapsulating drug molecules and have good permeability for membranes, and therefore can be used as excellent drug carriers.

The term "excipient" is an addition, besides the main drug, to a pharmaceutical formulation. It may also be referred to as an auxiliary material. For example, binders, fillers, disintegrants and lubricants in tablets; the matrix part in semisolid ointment and cream preparations; preservatives, antioxidants, corrigents, fragrances, cosolvents, emulsifiers, solubilizers, osmotic pressure regulators, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus facilitate the preparation of the tablet.

An HER2 antibody that "binds to the heterodimer binding site of HER2" binds to residues in the domain II (and optionally also binds to other domains in addition to the extracellular domain of HER2, such as residues in the domains I and III) and is capable of sterically hindering, at least to some extent, the formation of HER2-EGFR, HER2-HER3, or HER2-HER4 heterodimer. Franklin et al., Cancer Cell 5: 317-328(2004) showed the crystal structure of HER2-pertuzumab stored in the RCSB protein database (ID Code IS78) and illustrated an exemplary antibody that binds to a heterodimer binding site of HER2.

An antibody that "binds to the domain II of HER2" binds to residues in the domain II and optionally other domains of HER2, such as residues in the domains I and III. Preferably, an antibody that binds to the domain II binds to the junction between the domains I, II and III of HER2.

Herein, "pertuzumab" refers to an antibody comprising a light chain variable region and a heavy chain variable region having amino acid sequences set forth in SEQ ID NOs: 1 and 2, respectively. If pertuzumab is a complete antibody, it preferably comprises a light chain and a heavy chain having amino acid sequences set forth in SEQ ID NOs: 3 and 4, respectively. "Pertuzumab" refers to an antibody comprising a light chain variable region and a heavy chain variable region having amino acid sequences set forth in SEQ ID NOs: 1 and 2, respectively. "Trastuzumab" of the present disclosure comprises a light chain and a heavy chain having amino acid sequences set forth in SEQ ID NOs: 5 and 6, respectively. For the preparation method, reference is made to WO2006033700, and the relevant contents are incorporated herein for illustration.

## BRIEF DESCRIPTION OF THE DRAWINGS

FIG. 1 is a graph showing the change in tumor volume of N87/16-8 subcutaneous xenograft tumor model mice.
FIG. 2 is a graph showing the change in body weight ofN87/16-8 subcutaneous xenograft tumor model mice.
FIG. 3 is a graph showing the change in tumor volume of JIMIT-1 subcutaneous xenograft tumor model mice.
FIG. 4 is a graph showing the change in body weight of JIMIT-1 subcutaneous xenograft tumor model mice.

## DETAILED DESCRIPTION

The present disclosure is further described below with reference to examples, which are not intended to limit the scope of the present disclosure.

Experimental procedures without conditions specified in the examples of the present disclosure were generally conducted according to conventional conditions or according to conditions recommended by the manufacturers of the starting materials or commercial products. Reagents without specific origins indicated are commercially available conventional reagents.

The structures of compounds were determined by nuclear magnetic resonance (NMR) spectroscopy and/or mass spectrometry (MS). NMR shifts (δ) are given in $10^{-6}$ (ppm). NMR analysis was performed on a Bruker AVANCE-400 nuclear magnetic resonance instrument, with dimethyl sulfoxide-D6 (DMSO-$d_6$), chloroform-D ($CDCl_3$) and methanol-D4 ($CD_3OD$) as solvents and tetramethylsilane (TMS) as an internal standard. MS analysis was performed on an Agilent 1200/1290 DAD-6110/6120 Quadrupole MS liquid chromatography-mass spectrometry system (manufacturer: Agilent; MS model: 6110/6120 Quadrupole MS),
waters ACQuity UPLC-QD/SQD (manufacturer: waters; MS model: waters ACQuity Qda Detector/waters SQ Detector), and THERMO Ultimate 3000-Q Exactive (manufacturer: THERMO; MS model: THERMO Q Exactive).

High performance liquid chromatography (HPLC) analysis was performed using the following HPLC instruments: Agilent HPLC 1200DAD, Agilent HPLC 1200VWD and Waters HPLC e2695-2489.

Chiral HPLC analyses were performed on an Agilent 1260 DAD high performance liquid chromatograph.

Preparative high performance liquid chromatography was performed on Waters 2545-2767, Waters 2767-SQ Detecor2, Shimadzu LC-20AP and Gilson GX-281 preparative chromatographs.

Preparative chiral chromatography was performed on a Shimadzu LC-20AP preparative chromatograph.

The CombiFlash preparative flash chromatograph used was CombiFlash Rf200 (TELEDYNE ISCO).

Yantai Huanghai HSGF254 or Qingdao GF254 silica gel plates, 0.15-0.2 mm layer thickness, were adopted for thin-layer chromatography (TLC) analysis and 0.4-0.5 mm layer thickness for TLC separation and purification.

Yantai Huanghai silica gel of 200-300 mesh is generally used as a carrier in silica gel column chromatography.

Known starting materials described herein may be synthesized by using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can all be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Pressurized hydrogenation reactions were performed using a Parr 3916EKX hydrogenator and a Qinglan QL-500 hydrogenator, or an HC2-SS hydrogenator. Hydrogenation reactions generally involved 3 cycles of vacuumization and hydrogen purging.

Microwave reactions were performed on a CEM Discover-S 908860 microwave reactor. In the examples, a solution was an aqueous solution unless otherwise specified.

In the examples, reactions were conducted at room temperature, i.e., 20 °C to 30 °C, unless otherwise specified.

The eluent system for column chromatography and the developing solvent system for thin-layer chromatography used for compound purification included: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

### Example 1

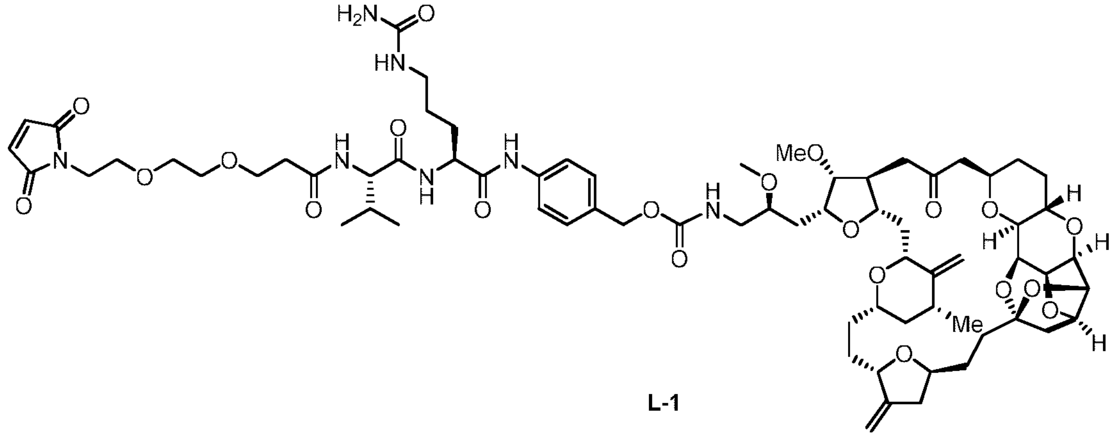

L-1

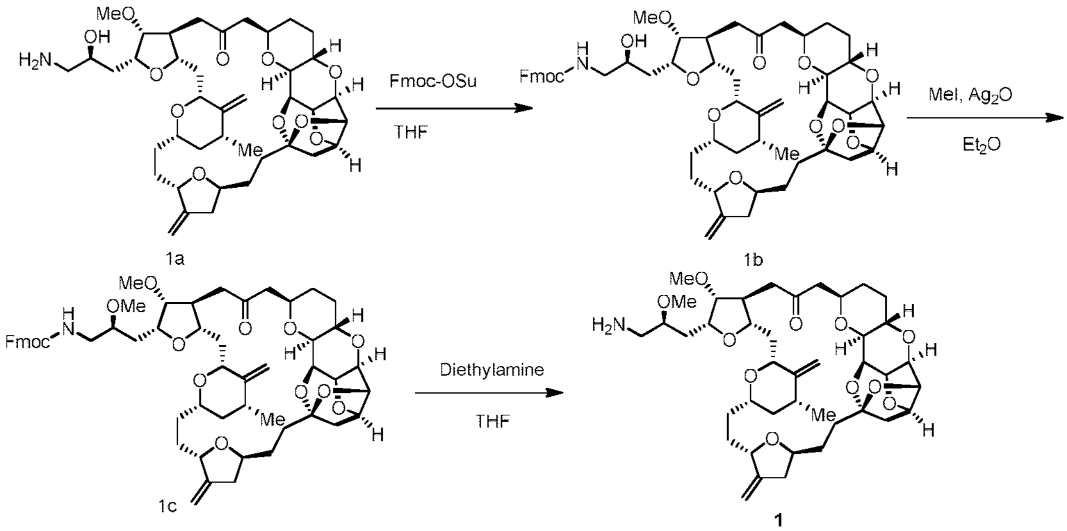


1a 1b 1c 1

Step 1: Preparation of compound **1b**

Compound **1a** (eribulin, prepared by referring to the method described in ZL201010236637.2) (72.91 mg, 0.1 mmol) was dissolved in 10 mL of tetrahydrofuran in an ice-water bath, followed by addition of Fmoc-OSu (*N-(9*-fluorenylmethoxycarbonyloxy)succinimide, 41 mg, 0.12 mmol). The reaction system was stirred at room temperature until the reaction was completed. The reaction system was concentrated under reduced pressure to give a crude product, which was used directly in the next step.

Step 2: Preparation of compound **1c**

The crude product of compound **1b** obtained in the previous step was dissolved in 10 mL of anhydrous ether, followed by addition of silver oxide (34.8 mg, 0.15 mmol) and iodomethane (28.4 mg, 0.2 mmol). The reaction system was stirred at room temperature until the reaction was completed. The reaction system was filtered and concentrated under reduced pressure to give a crude product, which was used directly in the next step.

Step 3: Preparation of compound **1**

The crude product of compound **1c** obtained in the previous step was dissolved in 10 mL of tetrahydrofuran, followed by addition of 2 mL of diethylamine. The reaction system was stirred at room temperature until the reaction was completed. The reaction system was concentrated under reduced pressure to obtain a crude product, which was purified by silica gel column chromatography (eluent: dichloromethane/ethyl acetate/petroleum ether) to give 3 mg of the target compound **1**.

LC/MS (ESI): m/z 744.2 $[M+H]^+$.

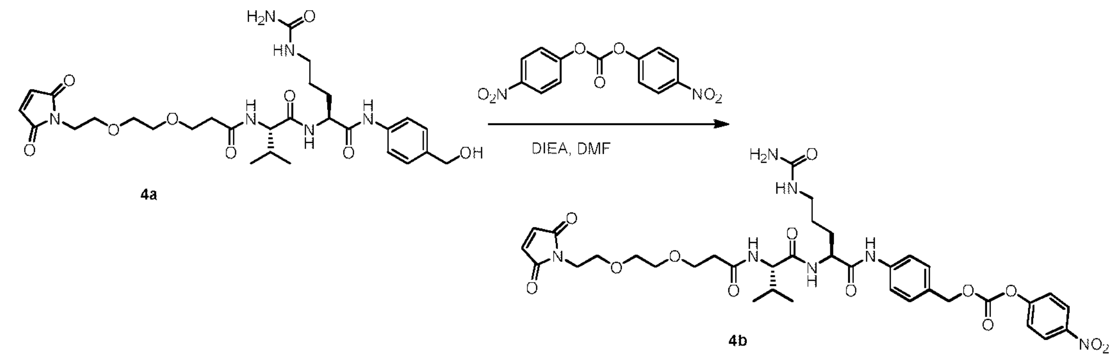


4a

4b

**4a** (50 mg, 0.08 mmol, prepared by referring to the method described in WO2017151979) was dissolved in 1.5 mL of *N,N*-dimethylformamide in an ice-water bath, followed by addition of DIPEA (*N,N*-diisopropylethylamine, 18 mg, 0.14 mmol) and bis(*p*-nitrophenyl)carbonate (49 mg, 0.16 mmol). The reaction system was stirred at room temperature for 10 min, and 20 mL of methyl *tert*-butyl ether was added. The reaction system was then stirred for 20 min, filtered, and dried to give 36 mg of a solid. LC/MS (ESI): m/z 784.1 $[M+H]^+$.

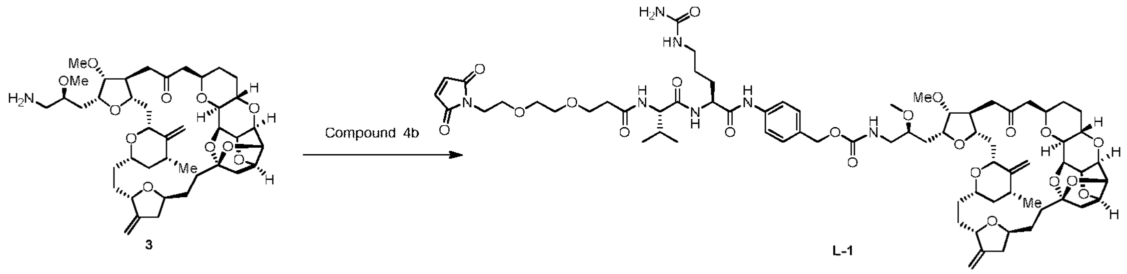


3

L-1

Compound **1** (13.5 mg, 0.018 mmol) was dissolved in 1.5 mL of DMF, followed by addition of DIPEA (7 mg, 0.054 mmol) and addition of compound **4b** (18 mg, 1.3 mmol) in portions. The reaction system was stirred until the reaction was substantially completed and then concentrated to give a crude product, which was separated by preparative HPLC to give 6.5 mg of compound L-1 (96.95% purity). LC/MS (ESI): m/z 1388.3 $[M+H]^+$.

**Example 2. ADC-001**

*N*-((2*R*,10*S*)-10-benzyl-2-cyclopropyl-1-(((1*S*,9*S*)-9-ethyl-5-fluoro-9-hydroxy-4-methyl-10, 13-dioxo-2,3,9, 1 0, 13, 15-hexahydro-1*H*, 12*H*-benzo[*de*]pyrano[3',4':6,7]indolizino[1,2-*b*]quinolin-1-yl)amino)-1,6,9,12,15-pentaoxo-3-oxa-5, 8,11,14-tetraazahexadec-16-yl)-6-(2,5-dioxo-2,5-dihydro-1*H*-pyrrol-1-yl)hexanamide **L-2A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5, 8,11,14-tetraazahexadec-16-yl)-6-(2,5-dioxo-2,5-dihydro-1*H*-pyrrol-1-yl)hexanamide **L-2B**

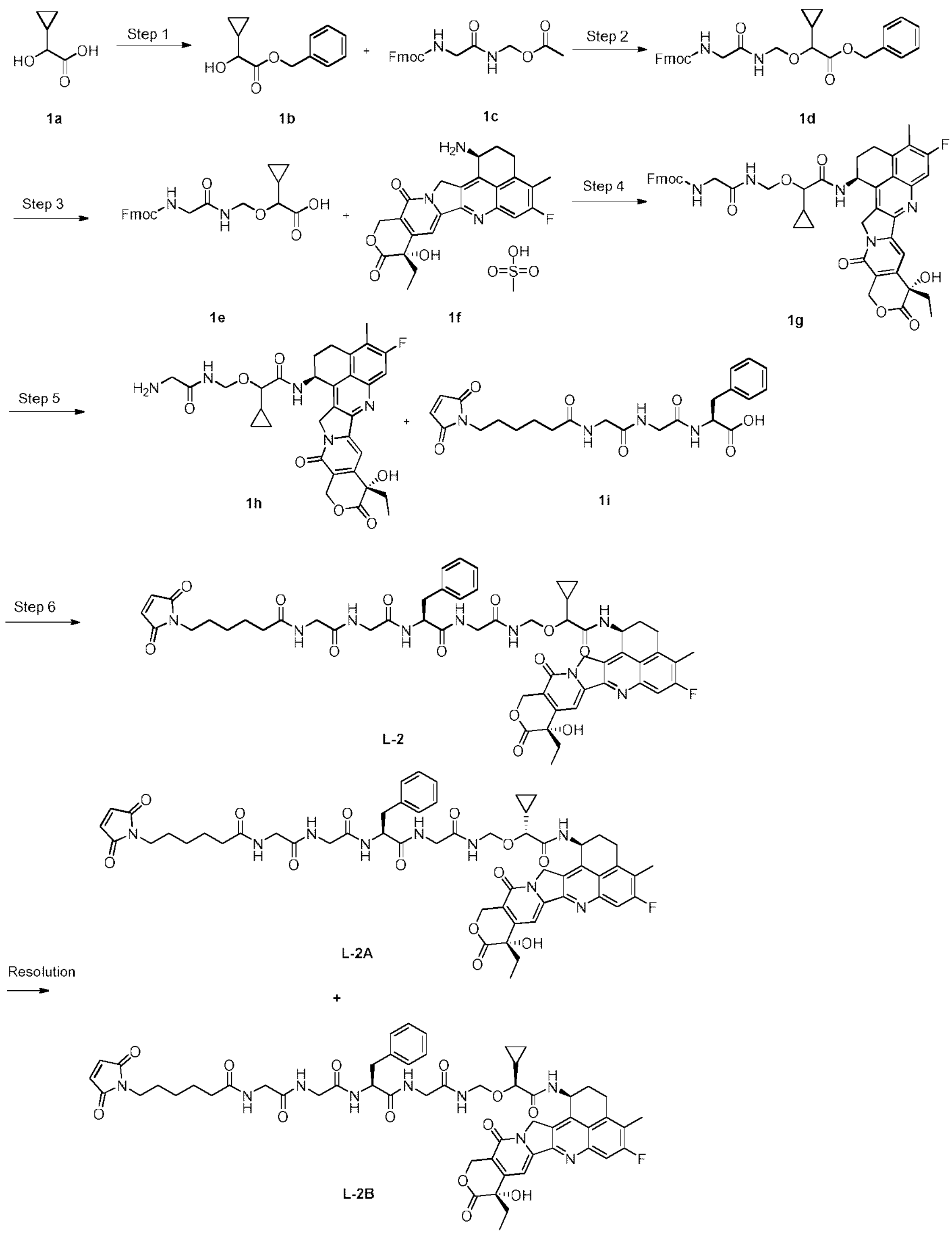


1a 1b 1c 1d 1e 1f 1g 1h 1i L-2 L-2A + L-2B

## Step 1

## Benzyl 2-cyclopropyl-2-hydroxyacetate **1b**

**1a** (1.3 g, 11.2 mmol; prepared according to the method disclosed in Patent Application "WO2013/106717") was dissolved in 50 mL of acetonitrile, and potassium carbonate (6.18 g, 44.8 mmol), benzyl bromide (1.33 mL, 11.2 mmol), and tetrabutylammonium iodide (413 mg, 1.1 mmol) were successively added. The reaction system was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **1b** (2 g, 86.9% yield).

Step 2

Benzyl 10-cyclopropyl-1-(9*H*-fluoren-9-yl)-3,6-dioxo-2,9-dioxa-4,7-diazaundecan-11-oate **1d**

To a reaction flask were added **1b** (120.9 mg, 0.586 mmol) and **1c** (180 mg, 0.489 mmol), followed by addition of 4 mL of tetrahydrofuran. The reaction system was purged with argon three times and cooled to 0-5 °C in an ice-water bath, followed by addition of potassium tert-butoxide (109 mg, 0.98 mmol). The ice bath was removed, and the resulting mixture was heated to room temperature and stirred for 40 min, followed by addition of 10 mL of ice water. The mixture was extracted with ethyl acetate (20 mL × 2) and chloroform (10 mL × 5), and the organic phases were combined and concentrated. The resulting residue was dissolved in 4 mL of dioxane, followed by addition of 2 mL of water, sodium bicarbonate (49.2 mg, 0.586 mmol), and 9-fluorenylmethyl chloroformate (126 mg, 0.49 mmol). The resulting mixture was stirred at room temperature for 2 h. 20 mL of water was added, and the mixture was extracted with ethyl acetate (10 mL × 3). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **1d** (48 mg, 19% yield).
MS m/z (ESI): 515.0 [M+1].

Step 3

10-cyclopropyl-1-(9*H*-fluoren-9-yl)-3,6-dioxo-2,9-dioxa-4,7-diazaundecan-11-oic acid **1e**

**1d** (20 mg, 0.038 mmol) was dissolved in 4.5 mL of a solvent mixture of tetrahydrofuran and ethyl acetate (V:V = 2:1), and palladium on carbon (12 mg, 10% loading, dry) was added. The reaction system was purged with hydrogen three times and stirred at room temperature for 1 h. The reaction system was filtered through celite, and the filter cake was rinsed with ethyl acetate. The filtrate was concentrated to give a crude product of the title product **1e** (13 mg), which was used directly in the next step without purification.
MS m/z (ESI): 424.9 [M+1].

Step 4

(9*H*-fluoren-9-yl)methyl (2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethyl)carbamate **1g**

To a reaction flask was added **1f** (10 mg, 18.8 μmol), followed by addition of 1 mL of N,N-dimethylformamide. The reaction system was purged with argon three times and cooled to 0-5 °C in an ice-water bath, followed by addition of a drop of triethylamine, the crude product of **9c** (13 mg, 30.6 μmol), and 4-(4,6-dimethoxy-1,3,5-triazin-2-yl)-4-methylmorpholinium chloride (16.9 mg, 61.2 μmol). The resulting reaction system was stirred in an ice bath for 40 min. 10 mL of water was added, and the mixture was extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin-layer chromatography with developing solvent system B to give the title product **1g** (19 mg, 73.6% yield).
MS m/z (ESI): 842.1 [M+1].

Step 5

2-((2-Aminoacetylamino)methoxy)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-b]quinolin-1-yl)acetamide **1h**

**1g** (19 mg, 22.6 μmol) was dissolved in 2 mL of dichloromethane, and 1 mL of diethylamine was added. The reaction system was stirred at room temperature for 2 h and concentrated under reduced pressure. 1 mL of toluene was added, and the resulting reaction system was concentrated under reduced pressure, and the procedures were repeated twice. The residue was slurried with 3 mL of *n*-hexane and left to stand. Then the supernatant was removed, and the solid was kept. The solid residue was concentrated under reduced pressure and dried using an oil pump to give a crude product of the title product **1h** (17 mg), which was used directly in the next step without purification.
MS m/z (ESI): 638.0 [M+18].

Step 6

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadec-16-yl)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadec-16-yl)-6-(2,5-dioxo-2,5-dihydro-1*H*-pyrrol-1-yl)hexanamide **9-B**

The crude product of **1h** (13.9 mg, 22.4 μmol) was dissolved in 0.6 mL of *N,N*-dimethylformamide. The reaction system was purged with argon three times and cooled to 0-5 °C in an ice-water bath, followed by addition of a solution of **1l** (21.2 mg, 44.8 μmol) in 0.3 mL of *N,N*-dimethylformamide and 4-(4,6-dimethoxy-1,3,5-triazin-2-yl)-4-methylmorpholinium chloride (18.5 mg, 67.3 μmol). The resulting reaction system was stirred in an ice bath for 10 min. Then the ice bath was removed, and the reaction system was heated to room temperature and stirred for 1 h to give compound **L-2**. The reaction system was purified by high performance liquid chromatography (separation conditions: chromatography column: XBridge Prep C18 OBD 5 μm 19 × 250 mm; mobile phase: A-water (10 mmol $NH_4OAc$), B-acetonitrile, gradient elution, flow rate: 18 mL/min). The corresponding fractions were collected and concentrated under reduced pressure to give the title products (L-2A: 2.4 mg, L-2B: 1.7 mg).

MS m/z (ESI): 1074.4 [M+1].

The one in compounds L-2A and L-2B that had a shorter retention time:
UPLC analysis: retention time: 1.14 min; purity: 85% (chromatography column: ACQUITY UPLC BEH C18 1.7 μm 2.1 × 50 mm; mobile phase: A-water (5 mmol $NH_4OAc$), B-acetonitrile).

$^1$H NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

The one in compounds L-2A and L-2B that had a longer retention time:
UPLC analysis: retention time: 1.16 min; purity: 89% (chromatography column: ACQUITY UPLC BEH C18 1.7 μm 2.1 × 50 mm; mobile phase: A-water (5 mmol $NH_4OAc$), B-acetonitrile).

$^1$H NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

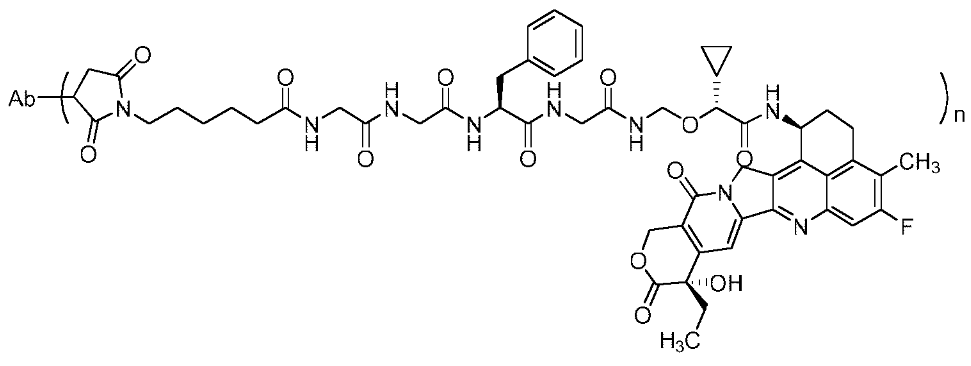

To an aqueous buffer solution of antibody pertuzumab in PBS (0.05 M aqueous PBS buffer solution at pH 6.5; 10.0 mg/mL, 1.50 mL, 101 nmol) was added a prepared aqueous solution of tris(2-carboxyethyl)phosphine hydrochloride (TCEP-HCl) (10 mM, 16.2 μL, 162 nmol) at 37 °C. The reaction system was shaken on a water bath shaker at 37 °C for 3 h, and then the reaction was stopped. The reaction system was cooled to 25 °C in a water bath.

The one in compounds L-2A and L-2B that had a shorter retention time (0.87 mg, 810 nmol) was dissolved in 66 μL of dimethyl sulfoxide, and the mixture was added to the reaction system described above. The resulting reaction system was shaken on a water bath shaker at 25 °C for 3 h, and then the reaction was stopped. The reaction system was desalted and purified through a Sephadex G25 gel column (elution phase: 0.05 M aqueous PBS buffer solution at

pH 6.5, containing 0.001 M EDTA) to give the title product in PBS buffer (1.16 mg/mL, 12.0 mL), which was refrigerated at 4 °C.

Mean calculated by RP-HPLC: n = 2.67.

**Example 3. ADC-002**

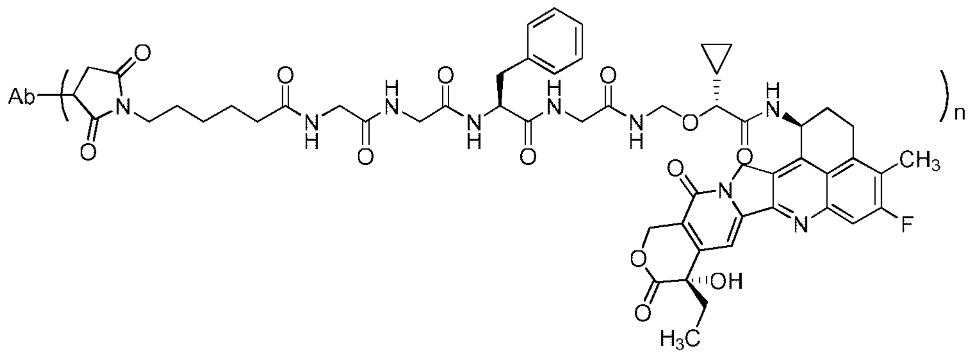

To an aqueous buffer solution of antibody pertuzumab in PBS (0.05 M aqueous PBS buffer solution at pH 6.5; 10.0 mg/mL, 1.50 mL, 101 nmol) was added a prepared aqueous solution of tris(2-carboxyethyl)phosphine hydrochloride (TCEP-HCl) (10 mM, 28.4 μL, 284 nmol) at 37 °C. The reaction system was shaken on a water bath shaker at 37 °C for 3 h, and then the reaction was stopped. The reaction system was cooled to 25 °C in a water bath.

The one in compounds L-2A and L-2B that had a shorter retention time (1.09 mg, 1015 nmol) was dissolved in 84 μL of dimethyl sulfoxide, and the mixture was added to the reaction system described above. The resulting reaction system was shaken on a water bath shaker at 25 °C for 3 h, and then the reaction was stopped. The reaction system was desalted and purified through a Sephadex G25 gel column (elution phase: 0.05 M aqueous PBS buffer solution at pH 6.5, containing 0.001 M EDTA) to give the title product in PBS buffer (1.06 mg/mL, 12.0 mL), which was refrigerated at 4 °C.

Mean calculated by RP-HPLC: n = 4.51.

**Example 4. ADC-003**

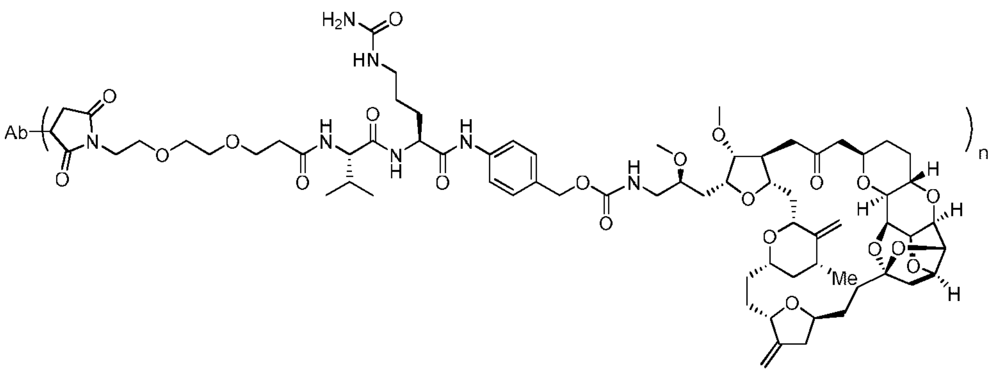

To an aqueous buffer solution of antibody pertuzumab in PBS (0.05 M aqueous PBS buffer solution at pH 6.5; 10.0 mg/mL, 1.43 mL, 97 nmol) was added a prepared aqueous solution of tris(2-carboxyethyl)phosphine hydrochloride (TCEP-HCl) (10 mM, 15.5 μL, 155 nmol) at 37 °C. The reaction system was shaken on a water bath shaker at 37 °C for 3 h, and then the reaction was stopped. The reaction system was cooled to 25 °C in a water bath.

Compound **L-1** (1.07 mg, 771 nmol) was dissolved in 50 μL of dimethyl sulfoxide, and the mixture was added to the reaction system described above. The resulting reaction system was shaken on a water bath shaker at 25 °C for 3 h, and then the reaction was stopped. The reaction system was desalted and purified through a Sephadex G25 gel

column (elution phase: 0.05 M aqueous PBS buffer solution at pH 6.5, containing 0.001 M EDTA) to give the title product in PBS buffer (1.21 mg/mL, 10.8 mL), which was refrigerated at 4 °C.

Mean calculated by CE-SDS: n = 2.04.

**Example 5. ADC-004**

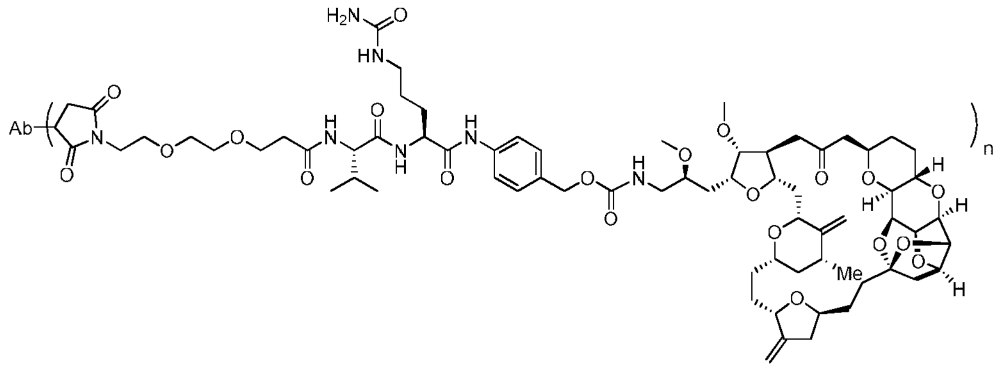

To an aqueous buffer solution of antibody pertuzumab in PBS (0.05 M aqueous PBS buffer solution at pH 6.5; 10.0 mg/mL, 1.43 mL, 97 nmol) was added a prepared aqueous solution of tris(2-carboxyethyl)phosphine hydrochloride (TCEP-HCl) (10 mM, 24.2 μL, 242 nmol) at 37 °C. The reaction system was shaken on a water bath shaker at 37 °C for 3 h, and then the reaction was stopped. The reaction system was cooled to 25 °C in a water bath.

Compound **L-1** (1.34 mg, 965 nmol) was dissolved in 60 μL of dimethyl sulfoxide, and the mixture was added to the reaction system described above. The resulting reaction system was shaken on a water bath shaker at 25 °C for 3 h, and then the reaction was stopped. The reaction system was desalted and purified through a Sephadex G25 gel column (elution phase: 0.05 M aqueous PBS buffer solution at pH 6.5, containing 0.001 M EDTA) to give the title product in PBS buffer (1.24 mg/mL, 9.8 mL), which was refrigerated at 4 °C.

Mean calculated by CE-SDS: n = 3.17.

**Example 6. ADC-005**

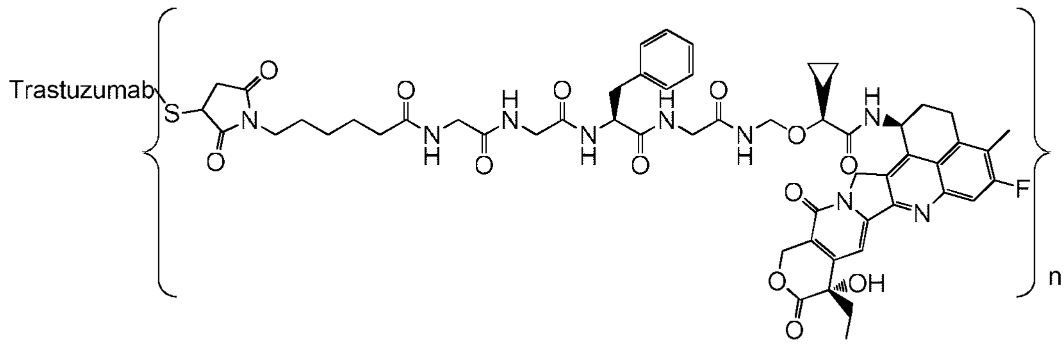

By referring to the method in Example 3, the one in compounds L-2A and L-2B that had a longer retention time and trastuzumab (prepared by referring to the method described in WO2006033700) were used as starting materials to prepare the target product in PBS buffer (1.24 mg/mL, 9.8 mL), which was refrigerated at 4 °C.

Mean calculated by CE-SDS: n = 6.

**Test Example 1. Inhibition of Proliferation of Human Breast Cancer BT-474 and Human Gastric Cancer NCI-N87, NCI-N87/16-8 and NCI-N87/8-2 Cells Cultured *In Vitro***

1.1 Drug information

| Sequence | Code for drug | Concentration (%) | Purity (%) |
|---|---|---|---|
| 1 | ADC-001 | 1.16 | 96.06 |
| 2 | ADC002 | 1.06 | 95.13 |
| 3 | ADC-003 | 1.21 | 98.18 |
| 4 | ADC-004 | 1.24 | 97.57 |
| 5 | ADC-005 | 5 | / |
| 6 | kadcyla | 10 | / |
| Note: the above samples were provided by Shanghai Hengrui Pharmaceutical Co., Ltd., wherein the pertuzumab was prepared by a method similar to that in WO2006033700; kadcyla: trastuzumab-maitansine conjugate. | | | |

1.2 Test cells

NCI-N87 and BT-474 cells were purchased from American Type Culture Collection (ATCC). T-DM1-resistant NCI-N87/16-8 and NCI-N87/8-2 cells induced by long-term effect of T-DM1 on NCI-N87 were constructed in this laboratory. Cells were cultured in an RPMI 1640/DMEM (1:1) medium containing 10% fetal bovine serum (FBS).

1.3 Instruments and reagents

RPMI 1640 and DMEM were purchased from Gibco BRL; FBS was purchased from Gibco; sulforhodamine B (SRB) was purchased from Sigma.

Microplate reader Synergy H4 was purchased from BioTek.

1.4 Experimental procedures

A certain number of cells in logarithmic growth phase were seeded into a 96-well culture plate. After the cells were subjected to adherence culture for 24 h, the drug at different concentrations (10000 ng/mL, 3000 ng/mL, 1000 ng/mL, 300 ng/mL, 100 ng/mL, 30 ng/mL, 10 ng/mL, 3 ng/mL, and 1 ng/mL) was added. After 120 h of drug treatment, the cells were immobilized with trichloroacetic acid. Then the cells were stained with an SRB solution; finally, a Tris solution was added to dissolve SRB. OD values at the wavelength of 510 nm were read using a microplate reader, and the cell growth inhibition rate was calculated according to the following formula:

Inhibition rate = (OD value of control well - OD value of dosing well)/OD value of control well $\times$ 100%

Half maximal inhibitory concentrations ($IC_{50}$ values) were calculated from inhibition rates at each concentration using GraphPad Prism 7 software.

Table 1. Inhibition ($IC_{50}$) of proliferation of HER2-positive tumor cells (ng/mL)

| | NCI-N87 | NCI-N87/16-8 | NCI-N87/8-2 | BT-474 |
|---|---|---|---|---|
| ADC-001 | ≈10000 | ≈10000 | ≈10000 | >10000 |
| ADC-001 | 1186 | 832.4 | 1257 | >10000 |
| ADC-003 | 13.0 | 104.5 | 55.4 | 8.7 |
| ADC-004 | 8.1 | 33.6 | 13.0 | 5.6 |
| ADC-005 | 391.3 | 235.7 | 96.5 | >10000 |
| ADC-005 | 26.3 | ≈10000 | ≈10000 | 77.3 |

**Test Example 2. *In Vitro* Cytotoxic Activity Screening of Compound 1**

1.1. Principle and method

In this experiment, the ATP content was determined using CTG to reflect the survival condition of the tumor cells. First, cells were seeded at different densities and cultured for 3 days and 5 days, and then the final culture condition was determined based on the $IC_{50}$ and the maximum inhibition rate. The killing effect of the toxin molecule was then assayed according to this condition.

1.2. Selection of cell strains

According to the purpose of the experiment, two disease models of breast cancer and NSCLC were selected, and three cell strains of SKBR3 tumor cells (HER2+, ATCC, Cat# HTB-30), MDA-MB-468 (HER2-, ATCC, Cat# HTB-132) and A549 (human non-small cell lung cancer cells, ATCC, Cat# CCL-185) were selected for screening.

1.3. Determination of cell culture conditions

1) Cell culturing: A549, SK-BR-3 and MDA-MB-468 cells were cultured in Ham's F-12K (Kaighn's) medium (Gibco, 21127030), McCoy's 5A medium (ThermoFisher, Cat# 16600108) and Leibovitz's L-15 medium (ThermoFisher, Cat# 11415-114) containing 10% FBS (Gibco, 10099-141), respectively.
2) Cell plating: A549 cells were digested with pancreatin, then the digestion was terminated with the above medium. The cells were counted, and $4.3 \times 10^5$, $7.2 \times 10^5$ and $11.5 \times 10^5$ cells were each added to a culture solution to give a final volume of 26 mL. 180 μL of cell suspension was added to each well in columns 2 to 11 of a 96-well plate (Corning, Cat# 3903) to give cell densities of 3K, 5K and 8K per well. Wells in column 12 were each filled with 200 μL of culture solution and the remaining wells were each filled with PBS. The SKBR3 and MDA-MB-468 cells were subjected to the same operations described above. Each sample was run in duplicate.
2) Drug preparation: stock solutions of positive control eribulin and the compound of the present disclosure were prepared in DMSO in a 96-well round bottom plate (Corning, Cat# 3788). 2 mM stock solution (stock solution diluted 10-fold in DMSO) was prepared in column 1 of drug preparation plate 1, and then 10-fold gradient dilution in DMSO was performed through column 10, and the wells in column 11 were filled with DMSO. 95 μL of corresponding culture solution was added into each well from column 2 to column 11 of the drug preparation plate 2, and 5 μL of solution was pipetted from column 2 to column 11 of drug preparation plate 1 to drug preparation plate 2. The solution was mixed well, and 20 μL of solution was pipetted and added into the plated cells. The cells were then cultured for 3 days and 5 days.
3) CTG assay (Cell Titer-GloTM, luminescent cell viability assay, Promega): the cell plates were taken out on day 3 and day 5 and equilibrated to room temperature. 90 μL of CTG was added into each well, and the mixture was reacted at room temperature for 10 min in the dark. The absorption value was read using a microplate reader and $IC_{50}$ was calculated.

1.4. Data results

Table 2

| | SKBR3 | | MDA-MB-468 | | A549 | |
|---|---|---|---|---|---|---|
| Compound | $IC_{50}$ (nM) | Maximum inhibition rate (%) | $IC_{50}$ (nM) | Maximum inhibition rate (%) | $IC_{50}$ (nM) | Maximum inhibition rate (%) |
| Eribulin | 0.7147 | 94.90 | 0.4819 | 87.47 | 0.6609 | 81.50 |
| Compound 1 | 0.2052 | 96.87 | 0.1827 | 88.01 | 0.5151 | 81.34 |

**Test Example 3. Pharmacokinetic Study of Compound 1**

1. Overview

Non-naive beagles were taken as test animals, and the plasma concentrations at different time points after intravenous administration of beagles with compound D-1 and eribulin were measured by using LC/MS/MS. The pharmacokinetic performance in beagles of the compound of the present disclosure was studied and the pharmacokinetic profile thereof was evaluated.

2. Test protocol

2.1. Test compounds

Compound D-1 and eribulin

2.2. Test animals

Six male beagles were divided into 2 groups of 3, purchased from Shanghai Medicilon Inc., and subjected to administration test.

2.3. Compound preparation

Compound D-1 was weighed out and dissolved in 5% (v/v) of DMSO, 20% (v/v) of PG and 20% (v/v) of PEG400, and then 55% (v/v) of normal saline was added to obtain a 0.25 mg/mL colorless and clear solution.
Eribulin was weighed out and dissolved in 5% (v/v) of DMSO, 20% (v/v) of PG and 20% (v/v) of PEG400, and then 55% (v/v) of normal saline was added to obtain a 0.25 mg/mL colorless and clear solution.

2.4. Administration

Beagles in one group were intravenously injected with compound D-1 at a dose of 0.5 mg/kg and at a volume of 2 mL/kg.
Beagles in another group were intravenously injected with eribulin at a dose of 0.5 mg/kg and at a volume of 2 mL/kg.

3. Procedures

The beagles were injected with compound D-1, and 1 mL of blood was collected before administration and at 5 min, 0.25 h, 0.5 h, 1.0 h, 2.0 h, 4.0 h, 8.0 h, 12.0 h and 24.0 h after administration. The collected blood samples were each placed in EDTA-K2 anticoagulant blood collection tubes, and the collected whole blood was placed on ice and centrifuged within 1 h to isolate the plasma (centrifugal force: 2200 g, centrifugal time: 10 min, 2-8 °C). Plasma samples were stored in a refrigerator at -80 °C prior to testing.
The beagles were injected with eribulin, and 1 mL of blood was collected before administration and at 5 min, 0.25 h, 0.5 h, 1.0 h, 2.0 h, 4.0 h, 8.0 h, 12.0 h and 24.0 h after administration. The collected blood samples were each placed in EDTA-K2 anticoagulant blood collection tubes, and the collected whole blood was placed on ice and centrifuged within 1 h to isolate the plasma (centrifugal force: 2200 g, centrifugal time: 10 min, 2-8 °C). Plasma samples were stored in a refrigerator at -80 °C prior to testing.
The content of the test compounds in beagle plasma after the injection was determined: 25 μL of beagle plasma at each time point after the administration was mixed with 50 μL (100 ng/mL) of internal standard solution camptothecin (Institute of Biological Products, National Institutes for Food and Drug Control) and 200 μL of acetonitrile. The mixture was vortexed for 5 min and centrifuged for 10 min (3700 rpm), and 3-4 μL of the supernatant of the plasma sample was taken for LC/MS/MS assay (API4000 triple quadrupole tandem mass spectrometer (No. 2), Applied Biosystems, USA; Shimadzu, LC-30AD ultra high performance liquid chromatography system, Shimadzu, Japan).

4. Pharmacokinetic parameters

Pharmacokinetic parameters for the compound of the present disclosure are shown in Table 3 below.

Table 3

| No. | Pharmacokinetic experiment for beagles | | | | | |
|---|---|---|---|---|---|---|
| | Plasma concentration C5min (ng /mL) | Area under curve AUC0-t (ng /mL*h) | Half-life T1/2 (h) | Retention time MRT (h) | Clearance CL (ml/min/kg) | Apparent volume of distribution Vz (ml/kg) |
| Compound 1 | 82.1 | 433 | 29.3 | 41.1 | 8.9 | 22039 |
| Eribulin | 217 | 106 | 3.5 | 3.7 | 78 | 15556 |

**Test Example 4: Therapeutic Effect of ADC-004 on Human Gastric Cancer NCI-N87/16-8 Nude Mouse Subcutaneous Xenograft Tumor**

1. Objective

The objective is to evaluate the therapeutic effect ofADC-004 on human gastric cancer NCI-N87/16-8 nude mouse subcutaneous xenograft tumor. 2. Test compound

ADC-004, diluted to the desired concentration with normal saline.

3. Cells

Human gastric cancer NCI-N87 cells were purchased from American Type Culture Collection. NCI-N87 was subjected to long-term induction with T-DM1 to develop resistance to T-DM1 and was named NCI-N87/16-8. The cells were cultured in a 10 cm petri dish with an RPMI 1640 medium (Gibco) containing 10% fetal bovine serum, penicillin and streptomycin in an incubator at 37 °C with 5% $CO_2$. Subculturing was performed 2-3 times a week. When the cells were in exponential growth phase, they were digested with pancreatin, collected, counted and inoculated.

4. Test animals

BALB/cJGpt-Foxn1$^{nu}$/Gpt mice, aged 4 weeks, female, purchased from Jiangsu GemPharmatech Co., Ltd. Production license No.: SCXK (Jiangsu) 2019-0009; animal certification No.: 202004958. Housing environment: SPF.

5. Test procedures

Each nude mouse was inoculated subcutaneously with $1 \times 10^7$ NCI-N87/16-8 cells, and after tumors grew to 100-150 $mm^3$, the animals were grouped according to tumor volume and body weight (D0). The mice were intravenously injected with 0.3 mg/mL and 0.6 mg/mL of ADC-004 at a volume of 10 mL/kg. The tumor volume and body weight were measured twice a week and the results were recorded.

6. Test indexes

The experimental index was to investigate the influence of the drug on the tumor growth, and the specific index was T/C% or tumor growth inhibition (TGI%).

Tumor diameters were measured twice a week with a vernier caliper and tumor volume (V) was calculated according to the following formula:

$$V = 1/2 \times a \times b^2$$ where a and b represent length and width, respectively.

where a and b represent length and width, respectively.

T/C (%) = $(T - T_0)/(C - C_0) \times 100$, where T and C represent the tumor volumes of animals at the end of the experiment; $T_0$ and $C_0$ represent the tumor volumes of animals at the beginning of the experiment.

$$\text{Tumor growth inhibition \% (TGI\%)} = 100 - T/C (\%).$$

When the tumor started to regress, tumor growth inhibition % (TGI%) = 100 - (T - $T_0$)/$T_0$ × 100.
If the volume of tumor shrinks compared with its initial volume, *i.e.,* T < $T_0$ or C < $C_0$, it is defined as partial regression (PR) of tumor; if the tumor completely disappears, it is defined as complete regression(CR) of tumor.
At the end of the experiment, at the experiment endpoint, or when the mean tumor volume in the solvent group reached 1500 $mm^3$, the animals were sacrificed by $CO_2$ anesthesia and dissected to give the tumors. The tumors were photographed.

7. Statistical analysis

Unless otherwise indicated, comparison between tumor volumes of the two groups was made by two-tailed Student's t-test, with P < 0.05 defined as statistically significant difference.

8. Results

On day 22 after single administration at doses of 3 mg/k and 6 mg/kg, ADC-004 could inhibit the growth of NCI-N87/16-8 cells in the nude mouse subcutaneous xenograft tumor model, and the tumor inhibition rates were 46.77% and 90.18%, respectively (*P* < 0.01 vs PBS control). The tumor-bearing mice were able to well tolerate ADC-004, and symptoms such as weight loss were not found.

9. Conclusion

ADC-004 can effectively inhibit the growth of human gastric cancer NCI-N87/16-8 cells in a nude mouse subcutaneous xenograft tumor model, and tumor-bearing mice can well tolerate ADC-004.

**Test Example 5: Therapeutic Effect of ADC-004 on JIMIT-1 Nude Mouse Subcutaneous Xenograft Tumor**

1. Test compound

ADC-004, diluted to the desired concentration with PBS.

2. Cells

Human breast cancer JIMIT-1 cells.

4. Test animals

Female Balb/c nude mice

5. Test procedures

BALB/c nude mice were each inoculated subcutaneously in the right dorsal part with JIMT1 cells 5 × $10^5$ JIMT1 cells. When the tumor volume reached 80-100 $mm^3$, the mice were randomly divided into groups of 6 according to the tumor volume and body weight, and the administration was started on the day of the grouping. HRA00092-C063-004 was diluted to 0.6 mg/mL with PBS and injected intravenously at a volume of 10 mL/kg, and the mice in the control group were injected intravenously with PBS at the same volume. The long and short diameters of the tumors were measured twice a week with a vernier caliper and the body weight of the mice was also measured.
Tumor volume (V) was calculated according to the following formula:

$$V = 1/2 \times a \times b^2$$

where a and b represent length and width, respectively.
T/C (%) = (T - $T_0$)/(C - $C_0$) × 100, where T and C represent the tumor volumes of animals at the end of the experiment; $T_0$ and $C_0$ represent the tumor volumes of animals at the beginning of the experiment.

$$\text{Tumor growth inhibition \% (TGI\%)} = 100 - \text{T/C (\%)}.$$

Unless otherwise indicated, comparison between tumor volumes of the two groups was made by two-tailed Student's t-test, with $P < 0.05$ defined as statistically significant difference.

6. Results

On day 24 after a single administration, ADC-004 could significantly inhibit the growth of JIMIT-1 cells in the nude mouse subcutaneous xenograft tumor model, and the tumor inhibition rate was 121.3%, and tumor regression was found in 5 mice (< 50 $mm^3$). The tumor-bearing mice were able to well tolerate ADC-004, and symptoms such as weight loss were not found.

## Claims

**1.** An antibody-drug conjugate having a structure of formula (I):

$$Ab\text{-}(L\text{-}D)_k \qquad (I)$$

or a pharmaceutically acceptable salt or solvate thereof;
wherein Ab is an HER2 antibody or an antigen-binding fragment thereof binding to a domain II of HER2, L is a linker covalently linking Ab to D, and k is 1 to 20;
-D is shown as the formula below:

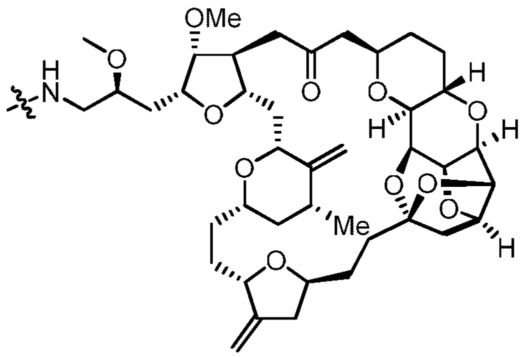

.

**2.** The antibody-drug conjugate according to claim 1, wherein k is selected from the group consisting of 1 to 10 and can be an integer or a decimal.

**3.** The antibody-drug conjugate according to claim 1 or 2, wherein the linker comprises a cleavable peptide moiety.

**4.** The antibody-drug conjugate according to claim 3, wherein the cleavable peptide moiety is capable of being cleaved by an enzyme, preferably being cleaved by a cathepsin, and further, the cathepsin is preferably cathepsin B.

**5.** The antibody-drug conjugate according to claim 3 or 4, wherein the linker comprises an amino acid unit, and the amino acid unit preferably comprises a peptide residue consisting of 2 to 7 amino acids selected from the group consisting of phenylalanine, glycine, valine, lysine, citrulline, serine, glutamic acid, and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe), and glycine-glycine-phenylalanine-glycine (Gly-Gly-Phe-Gly) (SEQ ID NO: 7).

**6.** The antibody-drug conjugate according to claim 1 or 2, wherein the linker comprises a cleavable sulfonamide moiety.

**7.** The antibody-drug conjugate according to any one of claims 1-3, wherein the linker comprises a cleavable disulfide moiety.

**8.** The antibody-drug conjugate according to claim 6 or 7, wherein the linker is capable of being cleaved under reduced conditions.

**9.** The antibody-drug conjugate according to any one of claims 1-8, wherein the linker comprises a spacer unit linking to D.

**10.** The antibody-drug conjugate according to claim 9, wherein the spacer unit comprises p-aminobenzyloxycarbonyl (pAB).

**11.** The antibody-drug conjugate according to claim 9, wherein the spacer unit comprises

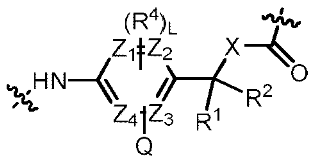

,

wherein $Z_1$-$Z_4$ are optionally selected from the group consisting of a carbon atom and a nitrogen atom; $R^4$ is selected from the group consisting of alkyl, cycloalkyl, aryl, and heteroaryl, and the alkyl, cycloalkyl, aryl, and heteroaryl are each independently optionally substituted with one or more substituents selected from the group consisting of alkyl, alkoxy, halogen, amino, cyano, nitro, hydroxy, hydroxyalkyl, cycloalkyl, heterocycloalkyl, aryl, and heteroaryl; $R^1$ and $R^2$ are each independently selected from the group consisting of hydrogen, $C_{1-6}$ alkyl, haloalkyl, and $C_{3-6}$ cycloalkyl, preferably hydrogen; or $R^1$ and $R^2$, together with the carbon atom to which they are attached, form $C_{3-6}$ cycloalkyl; X is selected from the group consisting of -O- and -NH-; L is selected from the group consisting of integers from 1 to 4;

Q is V-E, V-E provides a glycosidic bond cleavable by an intracellularly located glycosidase, and E is selected from the group consisting of -O-, -S- and $-NR^3-$, wherein $R^3$ is selected from the group consisting of hydrogen and methyl; further, V is selected from

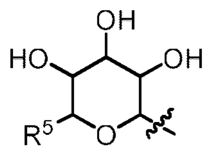

,

wherein $R^5$ is selected from the group consisting of -COOH and $CH_2OH$.

**12.** The antibody-drug conjugate according to any one of claims 1-11, wherein L-D is a chemical moiety represented by the formula below:

-Str- (Pep)-Sp-D, wherein Str is a stretcher unit covalently linking to Ab,
Sp is a spacer unit, and
Pep is selected from the group consisting of amino acid units.

**13.** The antibody-drug conjugate according to claim 12, wherein Str is selected from a chemical moiety represented by the formula below:

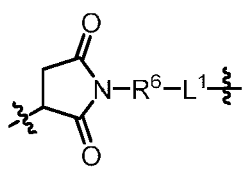

,

wherein $R^6$ is selected from the group consisting of -W-C(O)-, -C(O)-W-C(O)-, $-(CH_2CH_2O)_{p1}C(O)-$, $-(CH_2CH_2O)_{p1}CH_2C(O)-$, and - $(CH_2CH_2O)_{p1}CH_2CH_2C(O)-$, wherein W is selected from the group consisting of $C_{1-8}$ alkylene, $C_{1-8}$ alkylene-cycloalkyl, and linear heteroalkylene with 1 to 8 atoms, and the heteroalkylene comprises 1 to 3 heteroatoms selected from the group consisting of N, O, and S, wherein the $C_{1-8}$ alkylene, $C_{1-8}$ alkylene-cycloalkyl, and linear heteroalkylene are each independently optionally further substituted with one or more substituents selected from the group consisting of halogen, hydroxy, cyano, amino, alkyl, chloroalkyl, alkoxy, and cycloalkyl;

$L^1$ is selected from the group consisting of $-NR^7(CH_2CH_2O)_{p1}CH_2CH_2C(O)-$, $-NR^7(CH_2CH_2O)_{p1}CH_2C(O)-$, $-S(CH_2)_{p1}C(O)-$, $-(CH_2)_{p1}C(O)-$, and a chemical bond, wherein p1 is an integer from 1 to 20, and $L^1$ is preferably a chemical bond; p1 is an integer from 1 to 20, and $R^7$ is selected from the group consisting of hydrogen, alkyl, haloalkyl, and hydroxyalkyl.

**14.** The antibody-drug conjugate according to claim 13, wherein $R^6$ is selected from the group consisting of $C_{1-6}$ alkylene C(O)-, $-(CH_2-CH_2O)_2C(O)-$, $-(CH_2-CH_2O)_2CH_2C(O)-$, $-(CH_2-CH_2O)_2CH_2CH_2C(O)-$, $-(CH_2-CH_2O)_3C(O)-$, and $-(CH_2-CH_2O)_4C(O)-$.

**15.** The antibody-drug conjugate according to claim 13 or 14, wherein the linker L comprises: maleimide-$(PEG)_2$-Val-Cit, maleimide-$(PEG)_6$-Val-Cit, maleimide-$(PEG)_8$-Val-Cit, maleimide-$(PEG)_4$-$CH_2CH_2C(O)$-Val-lys, maleimide-$(CH_2)_5$-Val-Cit, maleimide-$(CH_2)_5$-Val-lys, maleimide-$(CH_2)_5$-Gly-Gly-Phe-Gly, maleimide-$(PEG)_2$-Ala-Ala-Asn, maleimide-$(PEG)_6$-Ala-Ala-Asn, maleimide-$(PEG)_8$-Ala-Ala-Asn, maleimide-$(PEG)_4$-triazole-$(PEG)_3$-sulfonamide, maleimide-$(PEG)_2$-$CH_2CH_2C(O)$-Val-lys, maleimide-$(PEG)_4$-triazole-$(PEG)_3$-sulfonamide, or Mal-$(PEG)_4$-triazole-$(PEG)_3$-disulfide.

**16.** The antibody-drug conjugate according to claim 12, wherein Str is selected from a chemical moiety represented by the formula below:

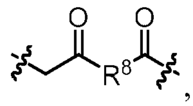

wherein $R^8$ is selected from the group consisting of $C_{1-10}$ alkylene, $C_{2-10}$ alkenylene, ($C_{1-10}$ alkylene)O-, $N(R^d)-(C_{2-6}$ alkylene)-$N(R^d)$, and $N(R^d)-(C_{2-6}$ alkylene); each $R^d$ is independently H or $C_{1-6}$ alkyl.

**17.** The antibody-drug conjugate according to any one of claims 1-15, wherein the antibody-drug conjugate is represented by the formulas below:

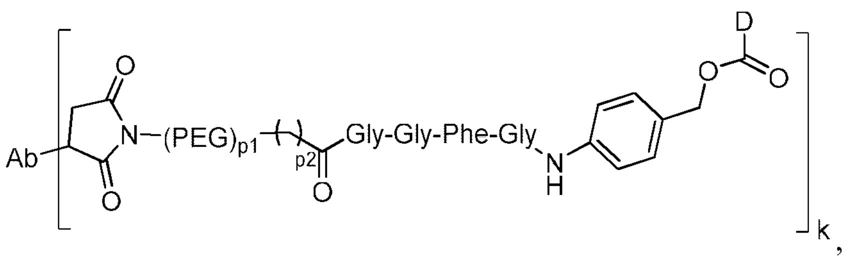

wherein k is selected from the group consisting of 1 to 10 and can be an integer or a decimal; p1 is selected from the group consisting of 2, 4, 6, and 8; p2 is selected from the group consisting of 0, 1, and 2;

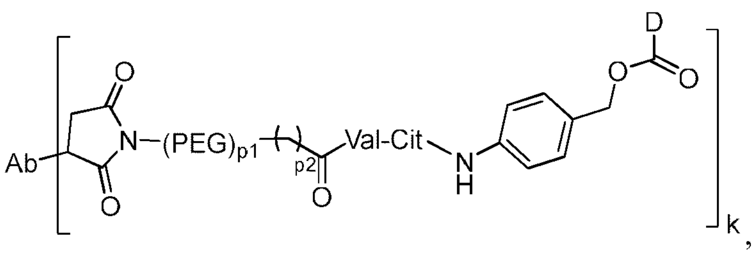

wherein k is selected from the group consisting of 1 to 10 and can be an integer or a decimal; p1 is selected from the group consisting of 2, 4, 6, and 8; p2 is selected from the group consisting of 0, 1, and 2;

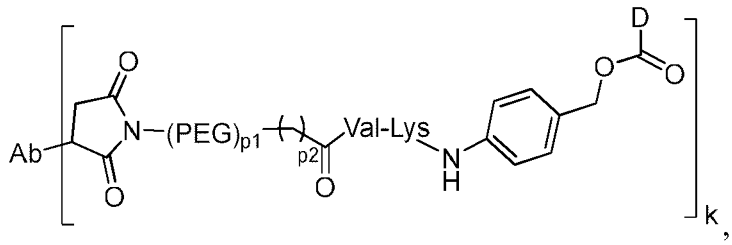

wherein k is selected from the group consisting of 1 to 10 and can be an integer or a decimal; p1 is selected from the group consisting of 2, 4, 6, and 8; p2 is selected from the group consisting of 0, 1, and 2;

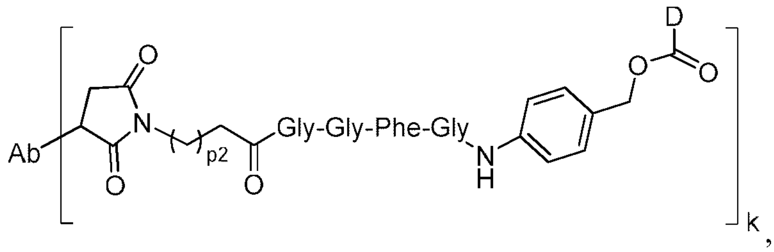

wherein k is selected from the group consisting of 1 to 10 and can be an integer or a decimal; p2 is selected from the group consisting of integers from 1 to 6;

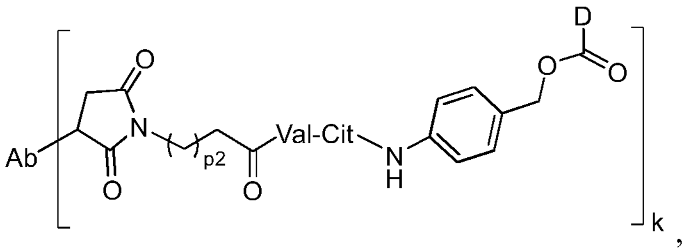

wherein k is selected from the group consisting of 1 to 10 and can be an integer or a decimal; p2 is selected from the group consisting of integers from 1 to 6;

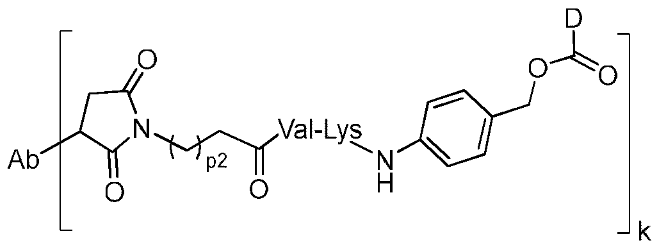

wherein k is selected from the group consisting of 1 to 10 and can be an integer or a decimal; p2 is selected from the group consisting of integers from 1 to 6;
Ab and D are as defined in claim 1.

**18.** The antibody-drug conjugate according to any one of claims 1-17, wherein the HER2 antibody comprises a light chain variable region and a heavy chain variable region having amino acid sequences set forth in SEQ ID NOs: 1 and 2, respectively, and preferably, the HER2 antibody comprises a light chain and a heavy chain having amino acid sequences set forth in SEQ ID NOs: 3 and 4, respectively.

**19.** The antibody-drug conjugate according to any one of claims 1-18, wherein the antibody-drug conjugate is represented

by the formulas below:

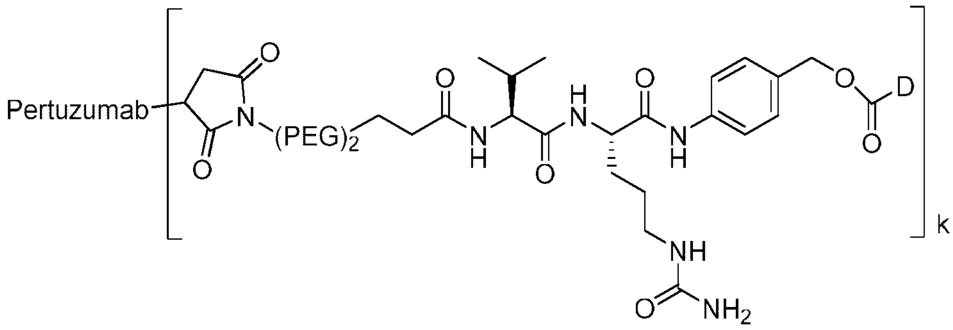

,

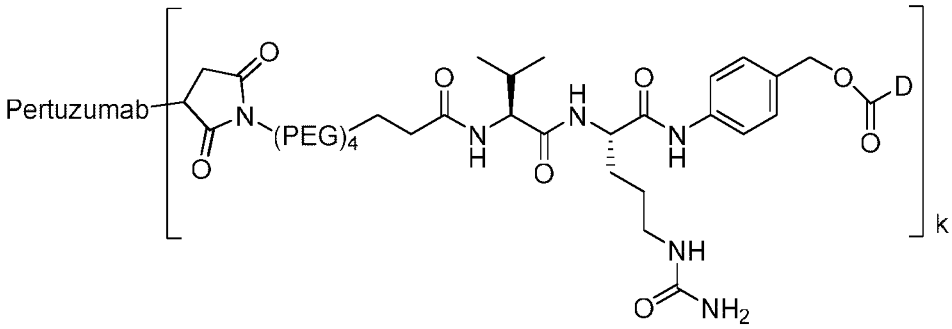

,

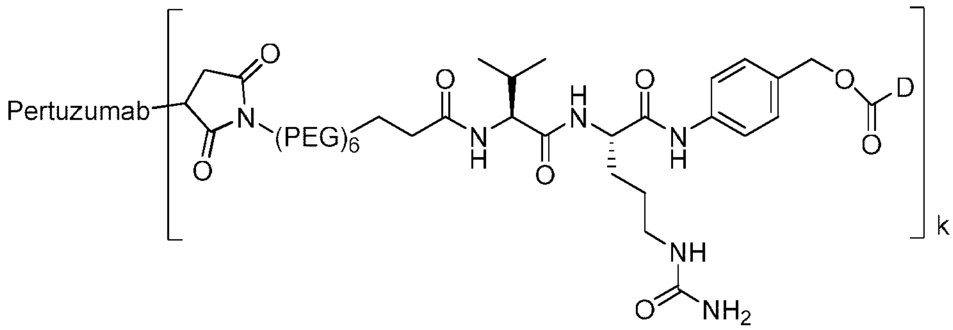

,

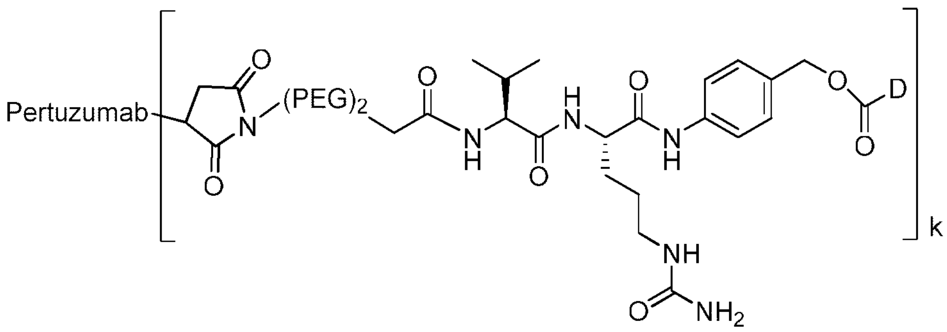

,

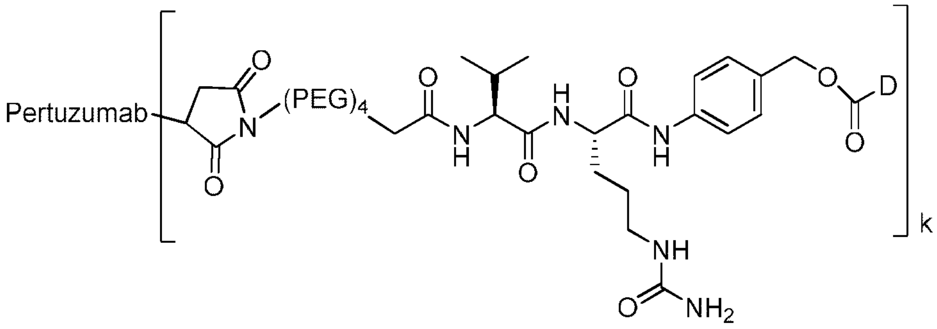

,

and

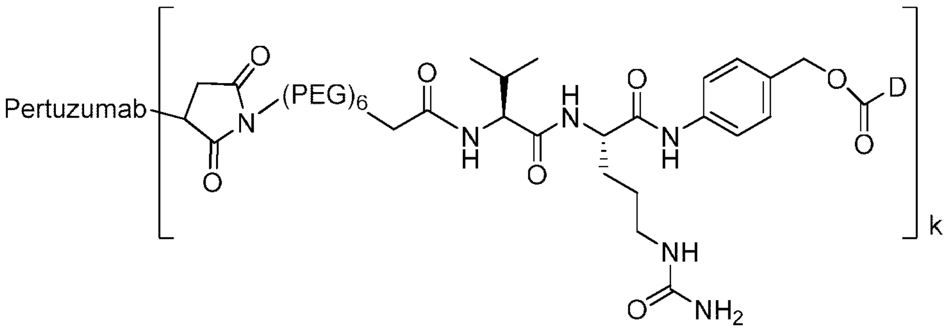

,

wherein k is selected from the group consisting of 1 to 10 and can be an integer or a decimal.

**20.** The antibody-drug conjugate according to any one of claims 1-19, wherein k is selected from the group consisting of 2.0 to 2.5, 2.5 to 3.5, and 3.5 to 5.0.

**21.** An isotopically substituted form of the antibody-drug conjugate according to any one of claims 1-20, wherein preferably, the isotopic substitution is a substitution with a deuterium atom.

**22.** A pharmaceutical composition, comprising:

a therapeutically effective amount of the antibody-drug conjugate according to any one of claims 1-20 or the isotopically substituted form according to claim 21, and
a pharmaceutically acceptable carrier, diluent, or excipient.

**23.** Use of the antibody-drug conjugate according to any one of claims 1-20, or the isotopically substituted form according to claim 21, or the pharmaceutical composition according to claim 22 in preparing a medicament for treating or preventing a tumor.

**24.** The use according to claim 23, wherein the tumor is cancer associated with expression of a domain II of HER2, and the cancer is preferably breast cancer, ovarian cancer, cervical cancer, uterine cancer, prostate cancer, kidney cancer, urinary tract cancer, bladder cancer, liver cancer, gastric cancer, endometrial cancer, salivary gland carcinoma, esophageal cancer, melanoma, neuroglioma, neuroblastoma, sarcoma, lung cancer, colon cancer, rectal cancer, colorectal cancer, leukemia, bone cancer, skin cancer, thyroid cancer, pancreatic cancer, and lymphoma.

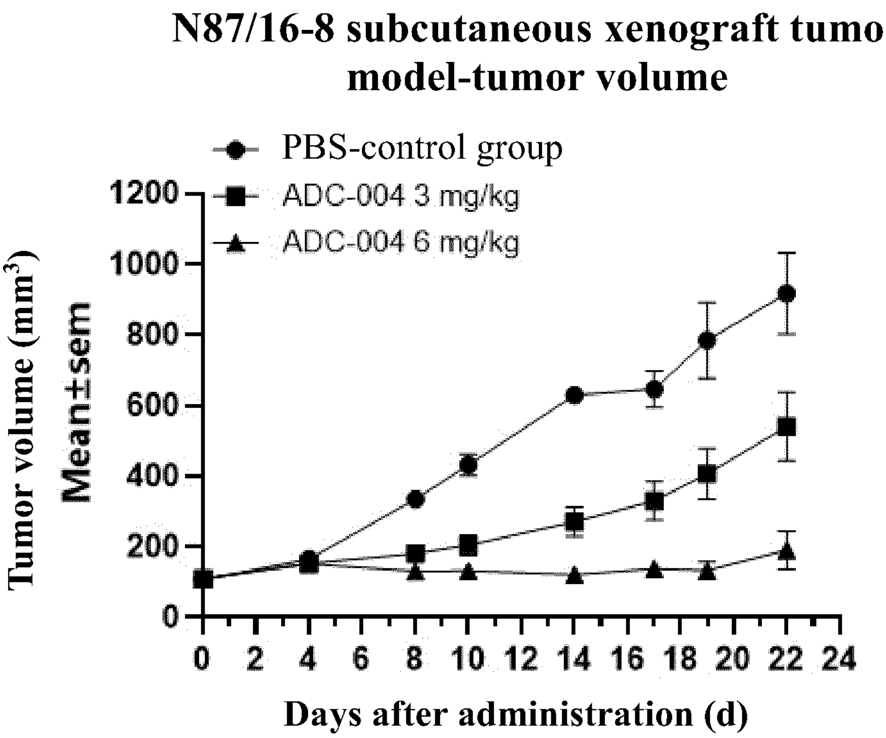
N87/16-8 subcutaneous xenograft tumor model-tumor volume
PBS-control group
ADC-004 3 mg/kg
ADC-004 6 mg/kg
Tumor volume (mm3) Mean±sem
1200
1000
800
600
400
200
0
0 2 4 6 8 10 12 14 16 18 20 22 24
Days after administration (d)

FIG. 1

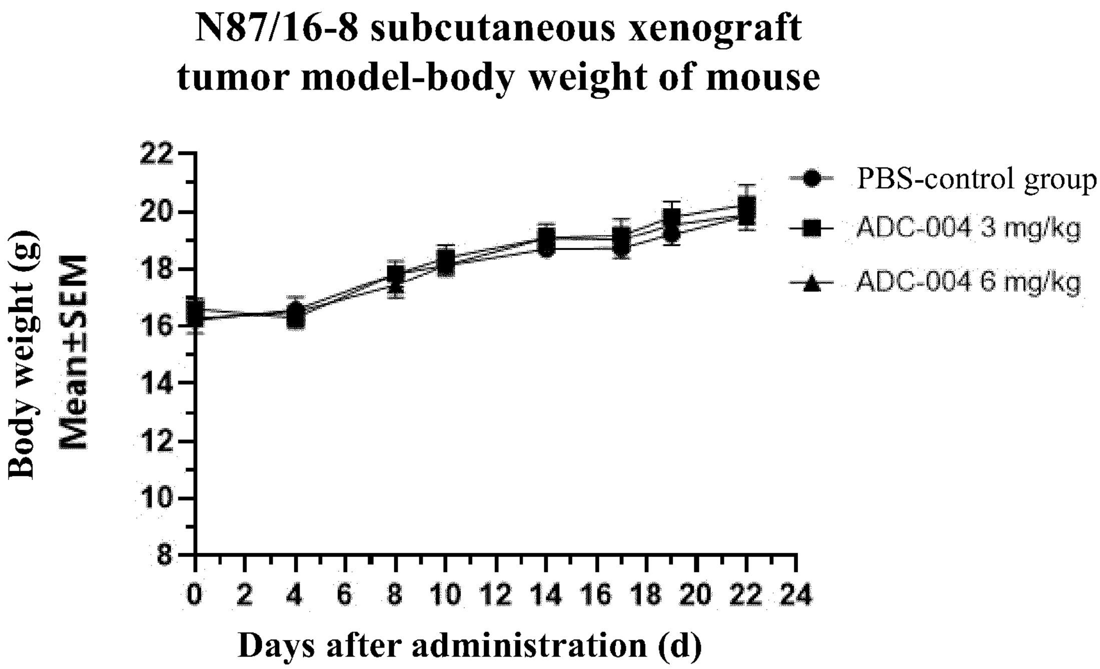
N87/16-8 subcutaneous xenograft tumor model-body weight of mouse
PBS-control group
ADC-004 3 mg/kg
ADC-004 6 mg/kg
Body weight (g) Mean±SEM
22
20
18
16
14
12
10
8
0 2 4 6 8 10 12 14 16 18 20 22 24
Days after administration (d)

FIG. 2

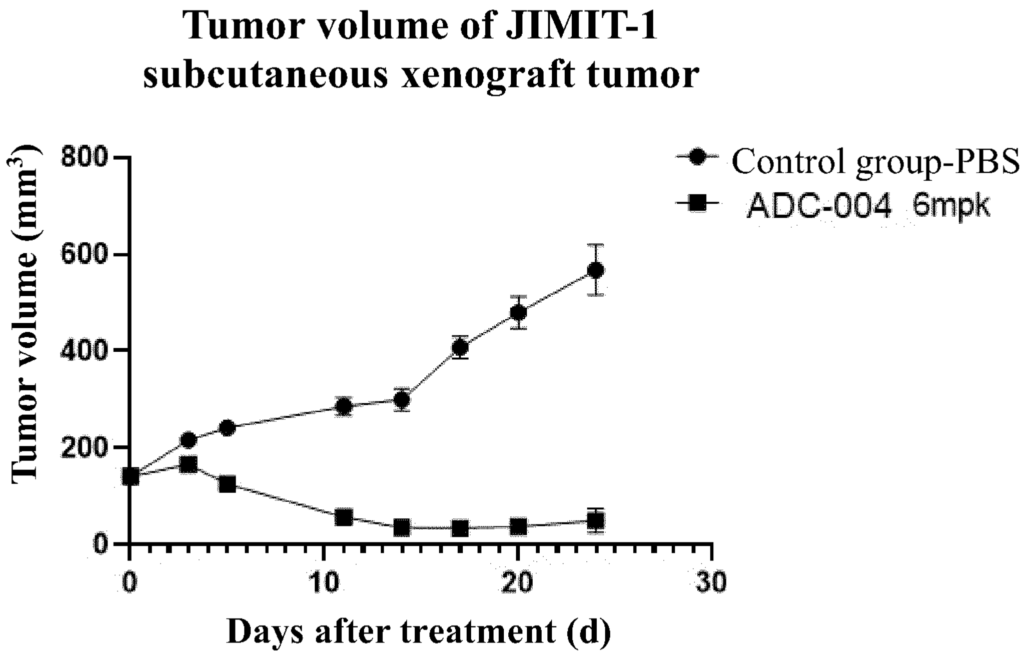
Tumor volume of JIMIT-1
subcutaneous xenograft tumor
Tumor volume (mm$^3$)
800
600
400
200
0
0
10
20
30
Days after treatment (d)
Control group-PBS
ADC-004 6mpk

FIG. 3

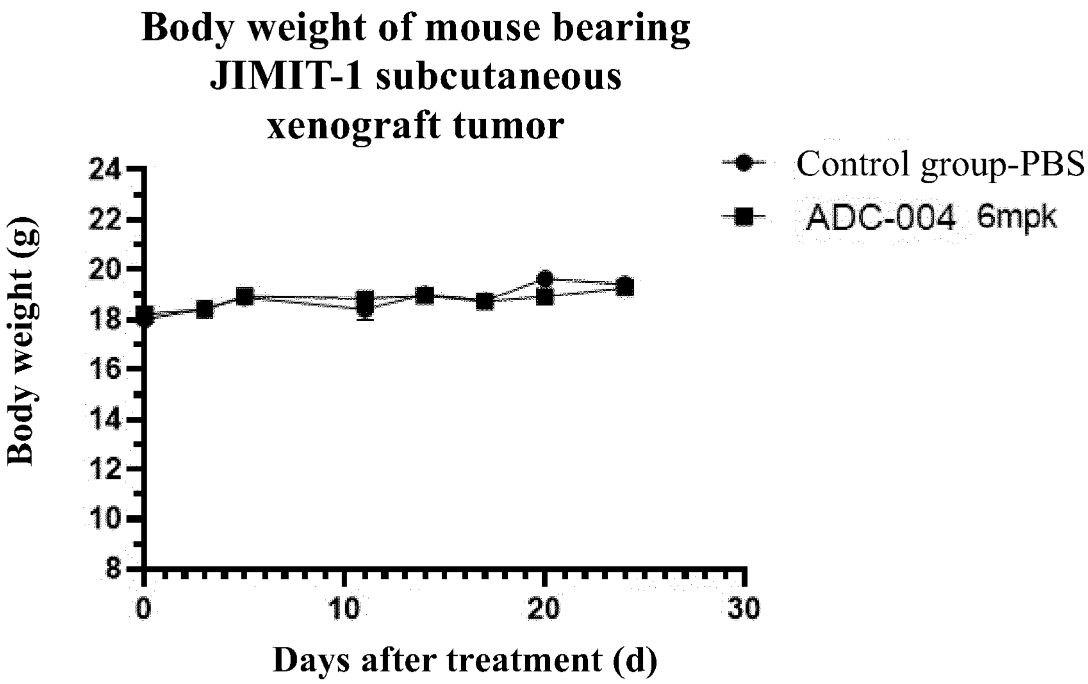
Body weight of mouse bearing
JIMIT-1 subcutaneous
xenograft tumor
Body weight (g)
24
22
20
18
16
14
12
10
8
0
10
20
30
Days after treatment (d)
Control group-PBS
ADC-004 6mpk

FIG. 4

**INTERNATIONAL SEARCH REPORT**

International application No. **PCT/CN2022/107479**

**A. CLASSIFICATION OF SUBJECT MATTER**

A61K 47/68(2017.01)i; C07K 16/28(2006.01)i; A61K 31/357(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNABS; CNTXT; SIPOABS; DWPI; CNKI; STNext: 孙星; 上海森辉; 上海盛迪, 上海恒瑞, 江苏恒瑞, 艾日布林; 抗体偶联药物; eribulin; antibody drug conjugate; ADC

**C. DOCUMENTS CONSIDERED TO BE RELEVANT**

| Category* | Citation of document, with indication, where appropriate, of the relevant passages | Relevant to claim No. |
|---|---|---|
| PX | WO 2022022508 A1 (TUOJIE BIOTECH (SHANGHAI) CO., LTD.) 03 February 2022 (2022-02-03)<br>claims 13-23 | 1-24 |
| X | CN 108883198 A (EISAI INC) 23 November 2018 (2018-11-23)<br>claims 1, 7, 11-13, 1882, 98, 142, and 146-147, paragraph 0597, and table 46 | 1-24 |
| X | WO 2021090062 A1 (EISAI R&D MANAGEMENT CO., LTD.) 14 May 2021 (2021-05-14)<br>claims 13-110 | 1-24 |
| X | WO 2021132166 A1 (EISAI R&D MANAGEMENT CO., LTD.) 01 July 2021 (2021-07-01)<br>claims 1-8 | 1-24 |
| X | CHENG, Xin et al. "MORAb-202, an Antibody–Drug Conjugate Utilizing Humanized Anti-human FRα Farletuzumab and the Microtubule-targeting Agent Eribulin, has Potent Antitumor Activity;"<br>*Molecular Cancer Therapeutics;*, Vol. 17, No. 12,, 01 December 2018 (2018-12-01),<br>et al. pages 2665–2675, abstract, table 1, and figure 1 | 1-24 |
| A | CN 1312804 A (EISAI CO., LTD.) 12 September 2001 (2001-09-12)<br>entire document | 1-24 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2022** | **26 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/107479**

| Patent document cited in search report | | | Publication date (day/month/year) | Patent family member(s) | | | Publication date (day/month/year) |
|---|---|---|---|---|---|---|---|
| WO | 2022022508 | A1 | 03 February 2022 | TW | 202214306 | A | 16 April 2022 |
| CN | 108883198 | A | 23 November 2018 | UA | 125024 | C2 | 29 December 2021 |
| | | | | CA | 3013791 | A1 | 08 September 2017 |
| | | | | JP | 2020019787 | A | 06 February 2020 |
| | | | | EP | 3824909 | A1 | 26 May 2021 |
| | | | | US | 2020297860 | A1 | 24 September 2020 |
| | | | | US | 2017252458 | A1 | 07 September 2017 |
| | | | | SG | 10202007520 W | A | 29 September 2020 |
| | | | | IL | 261428 | A | 31 October 2018 |
| | | | | LT | 3423105 | T | 10 September 2021 |
| | | | | AR | 121302 | A2 | 04 May 2022 |
| | | | | CO | 2018008667 | A2 | 31 August 2018 |
| | | | | SG | 11201806515 R | A | 27 September 2018 |
| | | | | BR | 112018067379 | A2 | 15 January 2019 |
| | | | | KR | 20220101204 | A | 19 July 2022 |
| | | | | RS | 62108 | B1 | 31 August 2021 |
| | | | | HU | E054726 | T2 | 28 September 2021 |
| | | | | KR | 20180115330 | A | 22 October 2018 |
| | | | | CN | 114191428 | A | 18 March 2022 |
| | | | | CN | 114191563 | A | 18 March 2022 |
| | | | | JP | 2020128413 | A | 27 August 2020 |
| | | | | CL | 2021000048 | A1 | 28 May 2021 |
| | | | | TW | 201800110 | A | 01 January 2018 |
| | | | | ES | 2880402 | T3 | 24 November 2021 |
| | | | | JP | 2019516664 | A | 20 June 2019 |
| | | | | PL | 3423105 | T3 | 25 October 2021 |
| | | | | HR | P20211125 | T1 | 15 October 2021 |
| | | | | MD | 3423105 | T2 | 31 October 2021 |
| | | | | EP | 3423105 | A1 | 09 January 2019 |
| | | | | AR | 107787 | A1 | 06 June 2018 |
| | | | | US | 2018193478 | A1 | 12 July 2018 |
| | | | | WO | 2017151979 | A1 | 08 September 2017 |
| | | | | IL | 292946 | A | 01 July 2022 |
| | | | | SI | 3423105 | T1 | 31 December 2021 |
| | | | | PH | 12018501847 | A1 | 15 May 2019 |
| | | | | PE | 20181953 | A1 | 17 December 2018 |
| | | | | AU | 2017225982 | A1 | 16 August 2018 |
| | | | | AR | 121301 | A2 | 04 May 2022 |
| | | | | DK | 3423105 | T3 | 26 July 2021 |
| | | | | MX | 2018010562 | A | 20 February 2019 |
| | | | | KR | 20220101203 | A | 19 July 2022 |
| | | | | CL | 2018002456 | A1 | 21 December 2018 |
| | | | | RU | 2018134331 | A | 02 April 2020 |
| | | | | PT | 3423105 | T | 19 July 2021 |
| | | | | RU | 2021125492 | A | 05 April 2022 |
| | | | | JP | 2021185176 | A | 09 December 2021 |
| | | | | CL | 2021000049 | A1 | 28 May 2021 |
| | | | | MA | 45280 | A | 09 January 2019 |
| | | | | CN | 114272389 | A | 05 April 2022 |
| WO | 2021090062 | A1 | 14 May 2021 | IL | 291643 | A | 01 May 2022 |

Form PCT/ISA/210 (patent family annex) (January 2015)

**INTERNATIONAL SEARCH REPORT**
**Information on patent family members**

International application No.
**PCT/CN2022/107479**

| Patent document cited in search report | Publication date (day/month/year) | Patent family member(s) | Publication date (day/month/year) |
|---|---|---|---|
| | | EP 4055053 A1 | 14 September 2022 |
| | | CA 3159541 A1 | 14 May 2021 |
| | | US 2021238304 A1 | 05 August 2021 |
| | | AU 2020380603 A1 | 02 June 2022 |
| | | KR 20220095197 A | 06 July 2022 |
| | | CO 2022007924 A2 | 30 June 2022 |
| | | TW 202132345 A | 01 September 2021 |
| WO 2021132166 A1 | 01 July 2021 | IL 293040 A | 01 July 2022 |
| | | KR 20220120586 A | 30 August 2022 |
| | | CA 3164797 A1 | 01 July 2021 |
| | | AU 2020414996 A1 | 14 July 2022 |
| | | JP WO2021132166 A1 | 01 July 2021 |
| CN 1312804 A | 12 September 2001 | AT 502932 T | 15 April 2011 |
| | | WO 9965894 A1 | 23 December 1999 |
| | | CA 2632433 A1 | 23 December 1999 |
| | | LU 91854 I2 | 17 October 2011 |
| | | US 6365759 B1 | 02 April 2002 |
| | | NO 20093217 L | 15 February 2001 |
| | | NO 2011026 I1 | 09 January 2012 |
| | | EP 2277873 A1 | 26 January 2011 |
| | | HK 1035534 A1 | 30 November 2001 |
| | | CA 2755266 A1 | 23 December 1999 |
| | | EP 2272840 A1 | 12 January 2011 |
| | | EP 1087960 A1 | 04 April 2001 |
| | | NZ 508597 A | 30 January 2004 |
| | | HU 0103357 A2 | 28 January 2002 |
| | | JP 2002518384 A | 25 June 2002 |
| | | NO 2022019 I1 | 03 June 2022 |
| | | US 6214865 B1 | 10 April 2001 |
| | | CY 1111516 T1 | 09 April 2014 |
| | | BR 9911326 A | 03 April 2001 |
| | | US 6469182 B1 | 22 October 2002 |
| | | IL 139960 D0 | 10 February 2002 |
| | | BE 2011C028 I2 | 24 August 2018 |
| | | AU 762998 C | 05 January 2000 |
| | | NO 20006316 D0 | 12 December 2000 |
| | | RU 2245335 C2 | 27 January 2005 |
| | | EP 2272839 A1 | 12 January 2011 |
| | | KR 20010083050 A | 31 August 2001 |
| | | DK 1087960 T3 | 14 June 2011 |
| | | PT 1087960 E | 17 June 2011 |
| | | CA 2335300 A1 | 23 December 1999 |
| | | ZA 200007159 B | 04 December 2001 |
| | | NO 2011018 I1 | 26 September 2011 |
| | | DE 69943296 D1 | 05 May 2011 |
| | | US 2011172446 A1 | 14 July 2011 |
| | | DE 122011100031 I1 | 15 December 2011 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050238649 A1
- US 5208020 A
- WO 2006033700 A
- WO 2017151979 A
- WO 2013106717 A


### Non-patent literature cited in the description


- **CHARI et al.** *Cancer Research,* 1992, vol. 52, 127-131
- *J. Biol. Chem,* 1968, vol. 243, 3558
- Chinese Pharmacopoeia 0542. 2015
- **KABAT, E. A. et al.** Sequences of Proteins of Immunological Interest. 1991
- **QUEEN et al.** *Proc. Natl. Acad. Sci. USA,* 1991, vol. 88, 2869
- **WINTER ; JONES et al.** *Nature,* 1986, vol. 321, 522
- **RIECHMANN et al.** *Nature,* 1988, vol. 332, 323-327
- **VERHOEYEN et al.** *Science,* 1988, vol. 239, 1534
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- **KABAT E.A. et al.** *Sequences of proteins of immunological interest,* 1991
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC M.P.** *Immunologist,* 1999, vol. 7, 132-136
- **LEFRANC, M.P. et al.** *Dev. Comp. Immunol.,* 2003, vol. 27, 55-77
- Epitope Mapping Protocols. Methods in Molecular Biology. 1996, vol. 66
- *immunoglobulin journal,* 2001, ISBN 012441351
- **FRANKLIN et al.** *Cancer Cell,* 2004, vol. 5, 317-328